# EUROPEAN PATENT APPLICATION

(11) **EP 4 488 294 A1**
(43) Date of publication of application: **08.01.2025**
(21) Application number: 23762874.8
(22) Date of filing: 28.02.2023
(51) Int. Cl.: C07K 16/32, A61K 39/395, A61P 35/00

(54) **HER3 BINDING PROTEIN AND USE THEREOF**

(30) Priority: 03.03.2022 CN 202210208312
(71) Applicant: Sichuan Kelun-Biotech Biopharmaceutical Co., Ltd., Chengdu, Sichuan 611138 (CN)
(72) Inventor: HU, Jiangjiang, Chengdu, Sichuan 611138 (CN); BAI, Yun, Chengdu, Sichuan 611138 (CN); LONG, Hu, Chengdu, Sichuan 611138 (CN); YUAN, Xiaoxi, Chengdu, Sichuan 611138 (CN)
(74) Representative: Meissner Bolte Partnerschaft mbB
(86) International application number: PCT/CN2023/078774
(87) International publication number: WO 2023/165475

(57) **Abstract**

Provided are an HER3 binding protein and use thereof. Specifically, provided are an anti-HER3 antibody or an antigen-binding fragment thereof, nucleic acid molecules encoding same, and a method for preparing same. The anti-HER3 antibody or the antigen-binding fragment thereof has high specificity and high affinity for HER3 and does not bind to EGFR, HER2, and HER4 of the same family. Further provided is use of the antibody or the antigen-binding fragment thereof in the treatment and diagnosis of disease.

## Description

### FIELD OF THE INVENTION

The present invention belongs to the field of therapeutic monoclonal antibodies, specifically relates to an antibody against HER3 as well as use of the antibody in treating and diagnosing diseases.

### BACKGROUND OF THE INVENTION

Human epidermal growth factor receptor 3 (HER3, also known as Erbb3) is a receptor tyrosine protein and belongs to the epidermal growth factor receptor (EGFR)/HER subfamily of receptor protein tyrosine kinase (RTK), and the subfamily consists of EGFR (HER1/Erbb1), HER2/Erbb2, HER3/Erbb3 and HER4/Erbb4. HER3 protein mainly comprises three domains: extracellular domain, transmembrane domain and intracellular domain, wherein the extracellular domain comprises four domains: D1, D2, D3 and D4.

EGFR and HER2 are recognized oncogenic RTKs that drive tumorigenesis in various types of solid tumors. A major role of HER3 in tumorigenesis is to serve as a scaffold protein, thereby enabling maximal induction of the PI3K/AKT pathway. HER3 contains a motif containing a cluster of 6 C-terminal tyrosines that, when phosphorylated, mimic the consensus PI3K/p85 binding site. Thus, the onco-drivers, EGFR, HER2, cMET and FGFR2 upstream can most efficiently bind to the PI3K/AKT pathway by forming heterodimers with HER3. Therefore, it is reasonable to expect that inhibition of HER3 activity could block the development of cancer in various systems driven by different RTKs. In addition to promoting tumor growth under non-stress conditions, HER3 has been found to be highly involved in conferring therapeutic resistance to a variety of targeted drugs, in which the targeted drugs include EGFR tyrosine kinase inhibitors, HER2 monoclonal antibodies such as trastuzumab mAb, and small molecule inhibitors of PI3K or AKT or MEK. Thus, HER3 holds promise as a cancer target to combat cancer tolerance issues.

There is currently no HER3-targeting antibody drug on the market. Therefore, it is urgent and necessary to develop a HER3-targeting antibody with higher specificity, lower toxicity, better clinical efficacy, and more convenient administration mode, which will provide patients with more medication options.

### CONTENT OF THE INVENTION

In the present application, the inventors developed fully human antibodies with high-affinity and excellent properties, which can specifically recognize/bind to HER3, but not to EGFR, HER2 and HER4 of the same family. The following inventions have been thus provided.

### Antibodies of the present invention

In one aspect, the present invention provides an antibody or antigen-binding fragment thereof that specifically binds to HER3, wherein the antibody or antigen-binding fragment thereof comprises the following complementarity determining regions (CDRs):
(a) CDR-H1, CDR-H2 and CDR-H3 contained in the heavy chain variable region (VH) set forth in SEQ ID NO: 1; and/or, CDR-L1, CDR-L2 and CDR-L3 contained in the light chain variable region (VL) set forth in SEQ ID NO: 2;
(b) CDR-H1, CDR-H2 and CDR-H3 contained in the heavy chain variable region (VH) set forth in SEQ ID NO: 3; and/or, CDR-L1, CDR-L2 and CDR-L3 contained in the light chain variable region (VL) set forth in SEQ ID NO: 4;
(c) CDR-H1, CDR-H2 and CDR-H3 contained in the heavy chain variable region (VH) set forth in SEQ ID NO: 5; and/or, CDR-L1, CDR-L2 and CDR-L3 contained in the light chain variable region (VL) set forth in SEQ ID NO: 6;
(d) CDR-H1, CDR-H2 and CDR-H3 contained in the heavy chain variable region (VH) set forth in SEQ ID NO: 7; and/or, CDR-L1, CDR-L2 and CDR-L3 contained in the light chain variable region (VL) set forth in SEQ ID NO: 8; or
(e) CDR-H1, CDR-H2, and CDR-H3 contained in the following heavy chain variable region (VH), and/or CDR-L1, CDR-L2 and CDR-L3 contained in the following light chain variable region (VL), wherein, the heavy chain variable region (VH) and/or the light chain variable region (VL) has at least one CDR containing a mutation compared to the heavy chain variable region and/or the light chain variable region described in any one of (a) to (d), and the mutation is a substitution, deletion or addition of one or several amino acids (e.g., a substitution, deletion or addition of 1, 2 or 3 amino acids).

In certain embodiments, the heavy chain variable region (VH) and/or the light chain variable region (VL) described in (e) above have at least one CDR containing a mutation compared to the corresponding heavy chain variable region and/or the light chain variable region described in any one of (a) to (d) respectively, in which the mutation is a substitution, deletion or addition of one or several amino acids.

In certain embodiments, the substitution is a conservative substitution.

In certain embodiments, the CDRs are defined according to the IMGT, Kabat or Chothia numbering system.

In certain embodiments, the HER3 comprises human HER3 and/or monkey HER3. In certain embodiments, the monkey is a Macaca mulatta.

In certain embodiments, the antibody or antigen-binding fragment thereof of the present invention comprises a heavy chain variable region (VH) and/or a light chain variable region (VL), wherein CDRs are defined by the IMGT numbering system:
(1a) a heavy chain variable region (VH) comprising the following 3 CDRs: CDR-H1 having a sequence set forth in SEQ ID NO: 9 or a variant thereof; CDR-H2 having a sequence set forth in SEQ ID NO: 10 or a variant thereof; CDR-H3 having a sequence set forth in SEQ ID NO: 11 or a variant thereof; and/or, a light chain variable region (VL) comprising the following 3 CDRs: CDR-L1 having a sequence set forth in SEQ ID NO: 18 or a variant thereof; CDR-L2 having a sequence set forth in SEQ ID NO: 19 or a variant thereof; CDR-L3 having a sequence set forth in SEQ ID NO: 20 or a variant thereof;
(1b) a heavy chain variable region (VH) comprising the following 3 CDRs: CDR-H1 having a sequence set forth in SEQ ID NO: 24 or a variant thereof; CDR-H2 having a sequence set forth in SEQ ID NO: 25 or a variant thereof; CDR-H3 having a sequence set forth in SEQ ID NO: 26 or a variant thereof; and/or, a light chain variable region (VL) comprising the following 3 CDRs: CDR-L1 having a sequence set forth in SEQ ID NO: 67 or a variant thereof; CDR-L2 having a sequence set forth in SEQ ID NO: 68 or a variant thereof; CDR-L3 having a sequence set forth in SEQ ID NO: 69 or a variant thereof;
(1c) a heavy chain variable region (VH) comprising the following 3 CDRs: CDR-H1 having a sequence set forth in SEQ ID NO: 37 or a variant thereof; CDR-H2 having a sequence set forth in SEQ ID NO: 38 or a variant thereof; CDR-H3 having a sequence set forth in SEQ ID NO: 39 or a variant thereof; and/or, a light chain variable region (VL) comprising the following 3 CDRs: CDR-L1 having a sequence set forth in SEQ ID NO: 32 or a variant thereof; CDR-L2 having a sequence set forth in SEQ ID NO: 33 or a variant thereof; CDR-L3 having a sequence set forth in SEQ ID NO: 34 or a variant thereof;
   or,
(1d) a heavy chain variable region (VH) comprising the following 3 CDRs: CDR-H1 having a sequence set forth in SEQ ID NO: 45 or a variant thereof; CDR-H2 having a sequence set forth in SEQ ID NO: 46 or a variant thereof; CDR-H3 having a sequence set forth in SEQ ID NO: 47 or a variant thereof; and/or, a light chain variable region (VL) comprising the following 3 CDRs: CDR-L1 having a sequence set forth in SEQ ID NO: 53 or a variant thereof; CDR-L2 having a sequence set forth in SEQ ID NO: 33 or a variant thereof; CDR-L3 having a sequence set forth in SEQ ID NO: 54 or a variant thereof;
wherein, the variant described in any one of (1a) to (1d) has a substitution, deletion or addition of one or several amino acids (e.g., a substitution, deletion or addition of 1, 2 or 3 amino acids) compared to the sequence from which it is derived; preferably, the substitution is a conservative substitution.

In certain embodiments, the antibody or antigen-binding fragment thereof of the present invention comprises a heavy chain variable region (VH) and/or a light chain variable region (VL), wherein CDRs are defined by the Kabat numbering system:
(2a) a heavy chain variable region (VH) comprising the following 3 CDRs: CDR-H1 having a sequence set forth in SEQ ID NO: 12 or a variant thereof; CDR-H2 having a sequence set forth in SEQ ID NO: 13 or a variant thereof; CDR-H3 having a sequence set forth in SEQ ID NO: 14 or a variant thereof; and/or, a light chain variable region (VL) comprising the following 3 CDRs: CDR-L1 having a sequence set forth in SEQ ID NO: 21 or a variant thereof; CDR-L2 having a sequence set forth in SEQ ID NO: 22 or a variant thereof; CDR-L3 having a sequence set forth in SEQ ID NO: 23 or a variant thereof;
(2b) a heavy chain variable region (VH) comprising the following 3 CDRs: CDR-H1 having a sequence set forth in SEQ ID NO: 27 or a variant thereof; CDR-H2 having a sequence set forth in SEQ ID NO: 28 or a variant thereof; CDR-H3 having a sequence set forth in SEQ ID NO: 29 or a variant thereof; and/or, a light chain variable region (VL) comprising the following 3 CDRs: CDR-L1 having a sequence set forth in SEQ ID NO: 70 or a variant thereof; CDR-L2 having a sequence set forth in SEQ ID NO: 71 or a variant thereof; CDR-L3 having a sequence set forth in SEQ ID NO: 69 or a variant thereof;
(2c) a heavy chain variable region (VH) comprising the following 3 CDRs: CDR-H1 having a sequence set forth in SEQ ID NO: 40 or a variant thereof; CDR-H2 having a sequence set forth in SEQ ID NO: 41 or a variant thereof; CDR-H3 having a sequence set forth in SEQ ID NO: 42 or a variant thereof; and/or, a light chain variable region (VL) comprising the following 3 CDRs: CDR-L1 having a sequence set forth in SEQ ID NO: 35 or a variant thereof; CDR-L2 having a sequence set forth in SEQ ID NO: 36 or a variant thereof; CDR-L3 having a sequence set forth in SEQ ID NO: 34 or a variant thereof;
   or
(2d) a heavy chain variable region (VH) comprising the following 3 CDRs: CDR-H1 having a sequence set forth in SEQ ID NO: 48 or a variant thereof; CDR-H2 having a sequence set forth in SEQ ID NO: 49 or a variant thereof; CDR-H3 having a sequence set forth in SEQ ID NO: 50 or a variant thereof; and/or, a light chain variable region (VL) comprising the following 3 CDRs: CDR-L1 having a sequence set forth in SEQ ID NO: 72 or a variant thereof; CDR-L2 having a sequence set forth in SEQ ID NO: 36 or a variant thereof; CDR-L3 having a sequence set forth in SEQ ID NO: 54 or a variant thereof;
wherein, the variant described in any one of (2a) to (2d) has a substitution, deletion or addition of one or several amino acids (e.g., a substitution, deletion or addition of 1, 2 or 3 amino acids) compared to the sequence from which it is derived; preferably, the substitution is a conservative substitution.

In certain embodiments, the antibody or antigen-binding fragment thereof of the present invention comprises a heavy chain variable region (VH) and/or a light chain variable region (VL), wherein CDRs are defined by the Chothia numbering system:
(3a) a heavy chain variable region (VH) comprising the following 3 CDRs: CDR-H1 having a sequence set forth in SEQ ID NO: 15 or a variant thereof; CDR-H2 having a sequence set forth in SEQ ID NO: 16 or a variant thereof; CDR-H3 having a sequence set forth in SEQ ID NO: 17 or a variant thereof; and/or, a light chain variable region (VL) comprising the following 3 CDRs: CDR-L1 having a sequence set forth in SEQ ID NO: 21 or a variant thereof; CDR-L2 having a sequence set forth in SEQ ID NO: 22 or a variant thereof; CDR-L3 having a sequence set forth in SEQ ID NO: 23 or a variant thereof;
(3b) a heavy chain variable region (VH) comprising the following 3 CDRs: CDR-H1 having a sequence set forth in SEQ ID NO: 30 or a variant thereof; CDR-H2 having a sequence set forth in SEQ ID NO: 31 or a variant thereof; CDR-H3 having a sequence set forth in SEQ ID NO: 29 or a variant thereof; and/or, a light chain variable region (VL) comprising the following 3 CDRs: CDR-L1 having a sequence set forth in SEQ ID NO: 70 or a variant thereof; CDR-L2 having a sequence set forth in SEQ ID NO: 71 or a variant thereof; CDR-L3 having a sequence set forth in SEQ ID NO: 69 or a variant thereof;
(3c) a heavy chain variable region (VH) comprising the following 3 CDRs: CDR-H1 having a sequence set forth in SEQ ID NO: 43 or a variant thereof; CDR-H2 having a sequence set forth in SEQ ID NO: 44 or a variant thereof; CDR-H3 having a sequence set forth in SEQ ID NO: 42 or a variant thereof; and/or, a light chain variable region (VL) comprising the following 3 CDRs: CDR-L1 having a sequence set forth in SEQ ID NO: 35 or a variant thereof; CDR-L2 having a sequence set forth in SEQ ID NO: 36 or a variant thereof; CDR-L3 having a sequence set forth in SEQ ID NO: 34 or a variant thereof;
   or
(3d) a heavy chain variable region (VH) comprising the following 3 CDRs: CDR-H1 having a sequence set forth in SEQ ID NO: 51 or a variant thereof; CDR-H2 having a sequence set forth in SEQ ID NO: 52 or a variant thereof; CDR-H3 having a sequence set forth in SEQ ID NO: 50 or a variant thereof; and/or, a light chain variable region (VL) comprising the following 3 CDRs: CDR-L1 having a sequence set forth in SEQ ID NO: 72 or a variant thereof; CDR-L2 having a sequence set forth in SEQ ID NO: 36 or a variant thereof; CDR-L3 having a sequence set forth in SEQ ID NO: 54 or a variant thereof;
wherein, the variant described in any one of (3a) to (3d) has a substitution, deletion or addition of one or several amino acids (e.g., a substitution, deletion or addition of 1, 2 or 3 amino acids) compared to the sequence from which it is derived; preferably, the substitution is a conservative substitution.

In certain embodiments, the antibody or antigen-binding fragment thereof of the present invention comprises:
(4a) a VH comprising the sequence set forth in SEQ ID NO: 1 or a variant thereof and/or a VL comprising the sequence set forth in SEQ ID NO: 2 or a variant thereof;
(4b) a VH comprising the sequence set forth in SEQ ID NO: 3 or a variant thereof and/or a VL comprising the sequence set forth in SEQ ID NO: 4 or a variant thereof;
(4c) a VH comprising a sequence as set forth in SEQ ID NO: 5 or a variant thereof and/or a VL comprising the sequence set forth in SEQ ID NO: 6 or a variant thereof;
   or
(4d) a VH comprising the sequence set forth in SEQ ID NO: 7 or a variant thereof and/or a VL comprising the sequence set forth in SEQ ID NO: 8 or a variant thereof;
wherein the variant has a sequence identity of at least 80%, at least 85%, at least 90%, at least 91%, at least 92%, at least 93%, at least 94%, at least 95%, at least 96%, at least 97%, at least 98%, at least 99%, or 100% compared to the sequence from which it is derived, or has a substitution, deletion, or addition of one or several amino acids (e.g., a substitution, deletion, or addition of 1, 2, 3, 4, or 5 amino acids) compared to the sequence from which it is derived; preferably, the substitution is a conservative substitution.

In certain embodiments, the antibody or antigen-binding fragment thereof having any one of the features (a), (1a), (2a), (3a), (4a) in the above embodiments further has a feature selected from the following:
(1) binding to D1 domain of HER3;
(2) binding to human or monkey HER3 with an EC50 of less than about 50 ng/mL, for example less than about 40 ng/mL, 30 ng/mL, 20 ng/mL, 15 ng/mL, 10 ng/mL, 9 ng/mL, 8 ng/mL, 7 ng/mL, 6 ng/mL, 5 ng/mL, 4 ng/mL or less; preferably, the EC50 is measured by ELISA;
(3) binding to human HER3 with a KD of less than about 50 nM, for example less than about 40 nM, 30 nM, 20 nM, 15 nM, 14 nM, 13 nM, 12 nM, 11 nM or lower; and/or, binding to monkey HER3 with a KD of less than about 100 nM, for example less than about 90 nM, 80 nM, 70 nM, 60 nM, 50 nM or lower; preferably, the KD is measured by biolayer interferometry (BLI) (e.g., ForteBio Octet^{®});
(4) not binding or substantially not binding to EGFR, HER2 and/or HER4; for example, measured by ELISA;
(5) having ADCC activity, such as inducing and killing HER3-expressing cells (e.g., tumor cells) through ADCC;
(6) inhibiting HER3-mediated signaling, such as inhibiting ligand-induced AKT phosphorylation, and/or inhibiting PI3K/AKT signaling;
(6) inducing HER3 internalization, for example determined by flow cytometry;
(7) inhibiting cell proliferation; and/or
(8) inhibiting tumor growth.

In certain embodiments, the antibody or antigen-binding fragment thereof having any one of the features (b), (1b), (2b), (3b) and (4b) of the above embodiments further has a feature selected from the following:
(1) binding to HER3 (e.g., human or monkey HER3) with an EC50 of less than about 50 ng/mL, such as less than about 40 ng/mL, 30 ng/mL, 20 ng/mL, 15 ng/mL, 14 ng/mL, 13 ng/mL, 12 ng/mL, 11 ng/mL, 10 ng/mL, 9 ng/mL, 8 ng/mL, 7 ng/mL, 6 ng/mL or less; preferably, the EC50 is measured by ELISA;
(2) not binding or substantially not binding to EGFR, HER2 and/or HER4; for example determined by ELISA;
(3) having ADCC activity, such as inducing and killing HER3-expressing cells (e.g., tumor cells) through ADCC;
(4) inhibiting HER3-mediated signaling, such as inhibiting ligand-induced AKT phosphorylation, and/or inhibiting PI3K/AKT signaling;
(5) inducing HER3 internalization, for example determined by flow cytometry;
(6) inhibiting cell (e.g., tumor cell) proliferation; and/or
(7) inhibiting tumor growth.

In certain embodiments, the antibody or antigen-binding fragment thereof having any one of the features (c), (1c), (2c), (3c) and (4c) in the above embodiments further has a feature selected from the following:
(1) binding to D1 domain of HER3;
(2) binding to human or monkey HER3 with an EC50 of less than about 50 ng/mL, such as less than about 40 ng/mL, 30 ng/mL, 20 ng/mL, 15 ng/mL, 10 ng/mL, 9 ng/mL, 8 ng/mL, 7 ng/mL or less; preferably, the EC50 is measured by ELISA;
(3) binding to human HER3 with a KD of less than about 50 nM, for example, less than about 40 nM, 30 nM or lower; and/or, binding to monkey HER3 with a KD of less than about 100 nM, for example, less than about 90 nM, 80 nM or lower; preferably, the KD is measured by biolayer interferometry (BLI) (e.g., ForteBio Octet^{®});
(4) not binding or substantially not binding to EGFR, HER2 and/or HER4; for example determined by ELISA;
(5) having ADCC activity, such as inducing and killing HER3-expressing cells (e.g., tumor cells) through ADCC;
(6) inhibiting HER3-mediated signaling, such as inhibiting ligand-induced AKT phosphorylation, and/or inhibiting PI3K/AKT signaling;
(6) inducing HER3 internalization, for example determined by flow cytometry;
(7) inhibiting cell proliferation; and/or
(8) inhibiting tumor growth.

In certain embodiments, the antibody or antigen-binding fragment thereof having any one of the features (d), (1d), (2d), (3d) and (4d) of the above embodiments further has a feature selected from the following:
(1) binding to D1 and D2 domains of HER3;
(2) binding to human, monkey or mouse HER3 with an EC50 of less than about 50 ng/mL, such as less than about 40 ng/mL, 30 ng/mL, 20 ng/mL, 15 ng/mL, 14 ng/mL, 13 ng/mL, 12 ng/mL, 11 ng/mL, 10 ng/mL, 9 ng/mL or less; preferably, the EC50 is measured by ELISA;
(3) binding to human HER3 with a KD of less than about 20 nM, such as less than about 15 nM, 10 nM, 9 nM, 8 nM or lower; and/or, binding to monkey HER3 with a KD of less than about 20 nM, such as less than about 15 nM, 10 nM, 9 nM, 8 nM, 7 nM, 6 nM or lower; preferably, the KD is measured by biolayer interferometry (BLI) (e.g., ForteBio Octet^{®});
(4) not binding or substantially not binding to EGFR, HER2 and/or HER4; for example determined by ELISA;
(5) having ADCC activity, such as inducing and killing HER3-expressing cells (e.g., tumor cells) through ADCC;
(6) inhibiting HER3-mediated signaling, such as inhibiting ligand-induced AKT phosphorylation, and/or inhibiting PI3K/AKT signaling;
(6) inducing HER3 internalization, for example determined by flow cytometry;
(7) inhibiting cell proliferation; and/or
(8) inhibiting tumor growth.

In certain embodiments, the antibody or antigen-binding fragment thereof described in any one of the above embodiments may comprise a constant region from or derived from a human immunoglobulin.

In certain embodiments, the antibody or antigen-binding fragment thereof has a heavy chain comprising a heavy chain constant region from or derived from a human immunoglobulin (e.g., IgGl, IgG2, IgG3 or IgG4). In certain embodiments, the antibody or antigen-binding fragment thereof has a heavy chain comprising a wild-type Fc region, or comprising a mutated or chemically modified Fc region, which has an altered effector function (such as enhanced ADCC activity) compared to the wild-type Fc region. In certain embodiments, the antibody or antigen-binding fragment thereof has a heavy chain comprising the sequence as set forth in SEQ ID NO: 63 or a variant thereof, in which the variant has a conservative substitution of up to 20 amino acids (e.g., a conservative substitution of up to 15, up to 10, or up to 5 amino acids; for example a conservative substitution of 1, 2, 3, 4 or 5 amino acids) compared thereto.

In certain embodiments, the antibody or antigen-binding fragment thereof has a light chain comprising a light chain constant region from or derived from a human immunoglobulin (e.g., κ or λ). In certain embodiments, the antibody or antigen-binding fragment thereof has a light chain comprising the sequence as set forth in SEQ ID NO: 65 or a variant thereof, in which the variant has a conservative substitution of up to 20 amino acids (e.g., a conservative substitution of up to 15, up to 10, or up to 5 amino acids; for example a conservative substitution of 1, 2, 3, 4 or 5 amino acids) compared thereto.

In certain embodiments, the antibody or antigen-binding fragment thereof of the present invention comprises:
(1) a heavy chain comprising a VH set forth in SEQ ID NO: 1 and a heavy chain constant region (CH) set forth in SEQ ID NO: 63, and/or, a light chain comprising a VL set forth in SEQ ID NO: 2 and a light chain constant region (CL) set forth in SEQ ID NO: 65;
(2) a heavy chain comprising a VH set forth in SEQ ID NO: 3 and a heavy chain constant region (CH) set forth in SEQ ID NO: 63, and/or, a light chain comprising a VL set forth in SEQ ID NO: 4 and a light chain constant region (CL) set forth in SEQ ID NO: 65;
(3) a heavy chain comprising a VH set forth in SEQ ID NO: 5 and a heavy chain constant region (CH) set forth in SEQ ID NO: 63, and/or, a light chain comprising a VL set forth in SEQ ID NO: 6 and a light chain constant region (CL) set forth in SEQ ID NO: 65;
   or,
(4) a heavy chain comprising a VH set forth in SEQ ID NO: 7 and a heavy chain constant region (CH) set forth in SEQ ID NO: 63, and/or, a light chain comprising a VL set forth in SEQ ID NO: 8 and a light chain constant region (CL) set forth in SEQ ID NO: 65.

In certain embodiments, the antibody or antigen-binding fragment thereof of the present invention comprises:
(1) a heavy chain comprising a VH set forth in SEQ ID NO: 1 and a heavy chain constant region (CH) set forth in SEQ ID NO: 63, and, a light chain comprising a VL set forth in SEQ ID NO: 2 and a light chain constant region (CL) set forth in SEQ ID NO: 65;
(2) a heavy chain comprising a VH set forth in SEQ ID NO: 3 and a heavy chain constant region (CH) set forth in SEQ ID NO: 63, and, a light chain comprising a VL set forth in SEQ ID NO: 4 and a light chain constant region (CL) set forth in SEQ ID NO: 65;
(3) a heavy chain comprising a VH set forth in SEQ ID NO: 5 and a heavy chain constant region (CH) set forth in SEQ ID NO: 63, and, a light chain comprising a VL set forth in SEQ ID NO: 6 and a light chain constant region (CL) set forth in SEQ ID NO: 65;
   or,
(4) a heavy chain comprising a VH set forth in SEQ ID NO: 7 and a heavy chain constant region (CH) set forth in SEQ ID NO: 63, and, a light chain comprising a VL set forth in SEQ ID NO: 8 and a light chain constant region (CL) set forth in SEQ ID NO: 65.

In certain embodiments, the antibody or antigen-binding fragment thereof described in any one of the above embodiments is a murine antibody, a chimeric antibody, a humanized antibody, or a fully human antibody.

In certain embodiments, the variable region of the antibody or antigen-binding fragment thereof described in any one of the above embodiments is a human variable region.

In certain embodiments, the antibody or antigen-binding fragment thereof described in any one of the above embodiments is selected from the group consisting of ScFv, Fab, Fab', (Fab')₂, Fab'-SH, Fv fragment, disulfide bond-linked Fv (dsFv), diabody, bispecific antibody and multispecific antibody.

In certain embodiments, the antibody or antigen-binding fragment thereof described in any one of the above embodiments bears a label. In certain embodiments, the antibody or antigen-binding fragment thereof bears a detectable label, such as an enzyme (e.g., horseradish peroxidase), a radionuclide, a fluorescent dye, a luminescent substance (e.g., a chemiluminescent substance), or a biotin.

### Derivatized antibody

The antibody or antigen-binding fragment thereof of the present invention can be derivatized, for example, linked to another molecule (e.g., another polypeptide or protein). Typically, derivatization (e.g., labeling) of an antibody or antigen-binding fragment thereof will not adversely affect its binding to HER3 (particularly human HER3). Accordingly, the antibody or antigen-binding fragment thereof of the present invention is also intended to include such derivatized forms. For example, the antibody or antigen-binding fragment thereof of the present invention can be functionally linked (by chemical coupling, genetic fusion, non-covalent linkage, or otherwise) to one or more other molecular moieties, such as another antibody (e.g., forming a bispecific antibody), detection reagent, pharmaceutical reagent, and/or protein or polypeptide (e.g., avidin or polyhistidine tag) capable of mediating the binding of the antibody or antigen-binding fragment to another molecule.

As one of the derivatives of the antibody, the present invention provides a conjugate, which comprises the antibody or antigen-binding fragment thereof of the present invention and a conjugating moiety.

In certain embodiments, the conjugating moiety is selected from detectable labels. The detectable label of the present invention can be any substance that can be detected by fluorescent, spectroscopic, photochemical, biochemical, immunological, electrical, optical or chemical means. Such labels are well known in the art, examples of which include, but are not limited to, enzymes (e.g., horseradish peroxidase, alkaline phosphatase, β-galactosidase, urease, glucose oxidase, etc.), radionuclides (e.g., 3H, 125I, 35S, 14C, or 32P), fluorescent dyes (e.g., fluorescein isothiocyanate (FITC), fluorescein, tetramethylrhodamine isothiocyanate (TRITC), phycoerythrin (PE), Texas Red, rhodamine, quantum dots or cyanine dye derivatives (e.g. Cy7, Alexa 750)), acridinium esters, magnetic beads (e.g. Dynabeads^{®}), calorimetric markers such as colloidal gold or colored glass or plastic (e.g., polystyrene, polypropylene, latex, etc.) beads, and biotin for binding to modified avidin (e.g., streptavidin) with the above-mentioned label. In certain embodiments, such labels can be adapted for use in immunological detection (e.g., enzyme-linked immunoassay, radioimmunoassay, fluorescent immunoassay, chemiluminescence immunoassay, etc.). In certain embodiments, the detectable label is selected from the group consisting of radioisotope, fluorescent substance, luminescent substance, colored substance or enzyme. In certain embodiments, the detectable label as described above can be attached to the antibody or antigen-binding fragment thereof of the present invention via a linker of various lengths to reduce potential steric hindrance.

In certain embodiments, the conjugating moiety is selected from therapeutic agents. In certain embodiments, the therapeutic agent is preferably an antitumor agent, such as a cytotoxic agent, cytokine, toxin or radionuclide.

In some embodiments, the conjugating moiety is selected from substances that can improve the biological properties (e.g., increasing serum half-life) of the antibody, for example it can be a chemical group such as polyethylene glycol (PEG), methyl or ethyl, or glycosyl.

As one of antibody derivatives, the present invention provides a multispecific antibody which comprises the antibody or antigen-binding fragment thereof of the present invention.

In certain embodiments, the multispecific antibody comprises the antibody or antigen-binding fragment thereof of the present invention as a first antigen-binding domain and further comprises at least one second antigen-binding domain directed against another target.

In certain embodiments, each antigen-binding domain of the multispecific antibody retains its original binding specificity.

In certain embodiments, the multispecific antibody is a bispecific antibody or a trispecific antibody or a tetraspecific antibody.

As one of the antibody derivatives, the present invention provides a chimeric antigen receptor, which comprises the antibody or antigen-binding fragment thereof of the present invention. In certain embodiments, the chimeric antigen receptor comprises the antibody or antigen-binding fragment thereof (e.g., ScFv) of the present invention as an extracellular antigen-binding domain that specifically binds to HER3, and a transmembrane domain and one or more intracellular T cell signaling domains. The present invention also provides a host cell (e.g., immune cell, such as T lymphocyte, NK cell) containing or expressing the chimeric antigen receptor.

### Preparation of antibody

The antibody of the present invention can be prepared by various methods known in the art, for example, by genetic engineering and recombination techniques. For example, DNA molecules encoding the heavy and light chain genes of the antibody of the present invention can be obtained by chemical synthesis or PCR amplification. The resulting DNA molecules can be inserted into an expression vector and then transfected into a host cell. Then, the transfected host cell can be cultured under specific conditions, and expresses the antibody of the present invention.

The antigen-binding fragment of the present invention can be obtained by hydrolyzing an intact antibody molecule (see, Morimoto et al., J. Biochem. Biophys. Methods 24:107-117 (1992) and Brennan et al., Science 229:81 (1985)). Alternatively, these antigen-binding fragments can also be produced directly by recombinant host cells (reviewed in Hudson, Curr. Opin. Immunol. 11: 548-557 (1999); Little et al., Immunol. Today, 21: 364-370 (2000)). For example, Fab' fragments can be obtained directly from host cells; Fab' fragments can be chemically coupled to form F(ab')₂ fragments (Carter et al., Bio/Technology, 10: 163-167 (1992)). In addition, Fv, Fab or F(ab')₂ fragments can also be directly isolated from the culture medium of recombinant host cells. Other techniques for preparing these antigen-binding fragments are well known to those of ordinary skill in the art.

Accordingly, in another aspect, the present invention provides an isolated nucleic acid molecule, which comprises a nucleotide sequence encoding the antibody or antigen-binding fragment thereof of the present invention, or heavy chain variable region and/or light chain variable region thereof. According to the codon degeneracy in the field, in some embodiments, the nucleotide sequence can be replaced according to the codon degeneracy. In certain embodiments, the nucleotide sequence is codon optimized.

In certain embodiments, the isolated nucleic acid molecule comprises a nucleic acid molecule encoding an antibody heavy chain variable region, and/or a nucleic acid molecule encoding an antibody light chain variable region, wherein:
the nucleic acid molecule encoding the antibody heavy chain variable region comprises: (i) a nucleotide sequence set forth in SEQ ID NO: 55, (ii) a sequence substantially identical to SEQ ID NO: 55 (e.g., a sequence having a sequence identity of at least about 85%, 90%, 95%, 99% or higher, or a sequence having a substitution of one or more nucleotides, as compared to SEQ ID NO: 55), or (iii) a degenerate sequence of the above (i) or (ii); and/or,
the nucleic acid molecule encoding the antibody light chain variable region comprises: (iv) a nucleotide sequence set forth in SEQ ID NO: 56, (v) a sequence substantially identical to SEQ ID NO: 56 (e.g., a sequence having a sequence identity of at least about 85%, 90%, 95%, 99% or higher, or a sequence having a substitution of one or more nucleotides, as compared to SEQ ID NO: 56), or (vi) a degenerate sequence of the above (iv) or (v).

In certain embodiments, the isolated nucleic acid molecule comprises a nucleic acid molecule encoding an antibody heavy chain variable region, and/or a nucleic acid molecule encoding an antibody light chain variable region, wherein:
the nucleic acid molecule encoding the antibody heavy chain variable region comprises: (i) a nucleotide sequence set forth in SEQ ID NO: 57, (ii) a sequence substantially identical to SEQ ID NO: 57 (e.g., a sequence having a sequence identity of at least about 85%, 90%, 95%, 99% or higher, or a sequence having a substitution of one or more nucleotides, as compared to SEQ ID NO: 57), or (iii) a degenerate sequence of the above (i) or (ii); and/or,
the nucleic acid molecule encoding the antibody light chain variable region comprises: (iv) a nucleotide sequence set forth in SEQ ID NO: 58, (v) a sequence substantially identical to SEQ ID NO: 58 (e.g., a sequence having a sequence identity of at least about 85%, 90%, 95%, 99% or higher, or a sequence having a substitution of one or more nucleotides, as compared to SEQ ID NO: 58), or (vi) a degenerate sequence of the above (iv) or (v).

In certain embodiments, the isolated nucleic acid molecule comprises a nucleic acid molecule encoding an antibody heavy chain variable region, and/or a nucleic acid molecule encoding an antibody light chain variable region, wherein:
the nucleic acid molecule encoding the antibody heavy chain variable region comprises: (i) a nucleotide sequence set forth in SEQ ID NO: 59, (ii) a sequence substantially identical to SEQ ID NO: 59 (e.g., a sequence having a sequence identity of at least about 85%, 90%, 95%, 99% or higher, or a sequence having a substitution of one or more nucleotides, compared to SEQ ID NO: 59), or (iii) a degenerate sequence of the above (i) or (ii); and/or,
the nucleic acid molecule encoding the antibody light chain variable region comprises: (iv) a nucleotide sequence set forth in SEQ ID NO: 60, (v) a sequence substantially identical to SEQ ID NO: 60 (e.g., a sequence having a sequence identity of at least about 85%, 90%, 95%, 99% or higher, or a sequence having a substitution of one or more nucleotides, compared to SEQ ID NO: 60), or (vi) a degenerate sequence of the above (iv) or (v).

In certain embodiments, the isolated nucleic acid molecule comprises a nucleic acid molecule encoding an antibody heavy chain variable region, and/or a nucleic acid molecule encoding an antibody light chain variable region, wherein:
the nucleic acid molecule encoding the antibody heavy chain variable region comprises: (i) a nucleotide sequence set forth in SEQ ID NO: 61, (ii) a sequence substantially identical to SEQ ID NO: 61 (e.g., a sequence having a sequence identity of at least about 85%, 90%, 95%, 99% or higher, or a sequence having a substitution of one or more nucleotides, as compared to SEQ ID NO: 61), or (iii) a degenerate sequence of the above (i) or (ii); and/or,
the nucleic acid molecule encoding the antibody light chain variable region comprises: (iv) a nucleotide sequence set forth in SEQ ID NO: 62, (v) a sequence substantially identical to SEQ ID NO: 62 (e.g., a sequence having a sequence identity of at least about 85%, 90%, 95%, 99% or higher, or a sequence having a substitution of one or more nucleotides, as compared to SEQ ID NO: 62), or (vi) a degenerate sequence of the above (iv) or (v).

In another aspect of the present invention, the present invention provides a vector (e.g., a cloning vector or an expression vector), which comprises the isolated nucleic acid molecule of the present invention. In certain embodiments, the vector of the present invention is, for example, plasmid, cosmid, phage, lentivirus, and the like. In certain embodiments, the vector is capable of expressing the antibody or antigen-binding fragment thereof of the present invention in a subject (e.g., a mammal, for example a human).

In certain embodiments, the vector comprises a first nucleotide sequence encoding a heavy chain or heavy chain variable region of the antibody or antigen-binding fragment thereof of the present invention and a second nucleotide sequence encoding a light chain or light chain variable region of the antibody or antigen-binding fragment thereof of the present invention, wherein the first nucleotide sequence and the second nucleotide sequence are present on the same or different vectors. When the first nucleotide sequence and the second nucleotide sequence are present on different vectors, the vector of the present invention comprises a first vector containing the first nucleotide sequence and a second vector containing the second nucleotide sequence.

In certain embodiments, the antibody or antigen-binding fragment thereof of the present invention can be used to construct a chimeric antigen receptor (CAR), and the chimeric antigen receptor comprises an extracellular antigen-binding domain (e.g., ScFv) that specifically binds to HER3, a transmembrane domain, and one or more intracellular T cell signaling domains. In such embodiments, the isolated nucleic acid molecule of the present invention may comprise a nucleotide sequence encoding a chimeric antigen receptor, in which the nucleotide sequence encoding the chimeric antigen receptor further comprises a nucleotide sequence encoding the antibody or antigen-binding fragment thereof (e.g., ScFv) of the present invention. In certain embodiments, the isolated nucleic acid molecule of the present invention encodes a chimeric antigen receptor comprising the antigen-binding fragment thereof (e.g., ScFv) of the antibody of the present invention.

In certain embodiments, the antibody or antigen-binding fragment thereof of the present invention can be used to construct a chimeric antigen receptor-modified immune cell, and the chimeric antigen receptor-modified immune cell comprises a chimeric antigen receptor (CAR) and an immune cell (e.g., T lymphocyte, NK cell).

In another aspect of the present invention, the present invention provides a host cell, which comprises the isolated nucleic acid molecule of the present invention or the vector of the present invention. The host cell can be a eukaryotic cell (e.g., mammalian cell, insect cell, yeast cell) or a prokaryotic cell (e.g., *E. coli*). Suitable eukaryotic cells include, but are not limited to, NS0 cell, Vero cell, Hela cell, COS cell, CHO cell, ExpiCHO cell, HEK293 cell, Expi293 cell, BHK cell, and MDCKII cell. Suitable insect cells include, but are not limited to, Sf9 cell. In certain embodiments, the host cell of the present invention is a mammalian cell, such as CHO cell (e.g., CHO-K1, CHO-S, CHO DXB11, ExpiCHO, CHO DG44 cell).

In certain embodiments, the host cell of the present invention may be a chimeric antigen receptor T cell (CAR-T). In such embodiments, the isolated nucleic acid molecule contained in the host cell may comprise a nucleotide sequence encoding a chimeric antigen receptor, and the nucleotide sequence encoding the chimeric antigen receptor further comprises a nucleotide sequence encoding the antibody or antigen-binding fragment thereof (e.g., ScFv) of the present invention. In certain embodiments, the host cell comprises an isolated nucleic acid molecule encoding a chimeric antigen receptor comprising the antigen-binding fragment thereof (e.g., ScFv) of the antibody of the present invention.

In another aspect of the present invention, there is provided a method for preparing the antibody or antigen-binding fragment thereof of the present invention, which comprises culturing the host cell of the present invention under conditions that allow the expression of the antibody or antigen-binding fragment thereof, and recovering the antibody or antigen-binding fragment thereof from a culture of the cultured host cell.

### Therapeutic application

Another aspect of the present invention provides a pharmaceutical composition, which comprises the antibody or antigen-binding fragment thereof, the nucleic acid molecule, the vector, the host cell, the conjugate, the multispecific antibody, or the chimeric antigen receptor or the host vector expressing the chimeric antigen receptor of the present invention, and a pharmaceutically acceptable carrier and/or excipient.

In certain embodiments, the pharmaceutical composition of the present invention comprises the antibody or antigen-binding fragment thereof of the present invention, and a pharmaceutically acceptable carrier and/or excipient.

In certain embodiments, the pharmaceutical composition of the present invention comprises the vector or the host cell of the present invention, and a pharmaceutically acceptable carrier and/or excipient. In such embodiments, the isolated nucleic acid molecule contained in the vector comprises a nucleotide sequence encoding a chimeric antigen receptor, and the nucleotide sequence encoding the chimeric antigen receptor further comprises a nucleotide sequence encoding the antibody or antigen-binding fragment thereof (e.g., ScFv) of the present invention; and the host cell comprises the isolated nucleic acid molecule or vector as described above. In certain embodiments, the isolated nucleic acid molecule encodes a chimeric antigen receptor comprising the antigen-binding fragment (e.g., ScFv) of the antibody of the present invention. In certain embodiments, the host cell is an immune cell, such as T cell. In certain embodiments, the host cell is a chimeric antigen receptor T cell (CAR-T).

In certain embodiments, the pharmaceutical composition may further comprise an additional pharmaceutically active agent. In certain embodiments, the additional pharmaceutically active agent is a drug having antitumor activity. In certain embodiments, the additional pharmaceutically active agent is selected from the group consisting of EGFR inhibitor, HER2 inhibitor, HER3 inhibitor, HER4 inhibitor, IGFR-1 inhibitor, mTOR inhibitor, PI3 kinase inhibitor, c-met or VEGF inhibitor, chemotherapy drug, or any combination thereof. In certain embodiments, the antibody or antigen-binding fragment thereof of the present invention and the additional pharmaceutically active agent are provided as separate components or as mixed components. Accordingly, the antibody or antigen-binding fragment thereof of the present invention and the additional pharmaceutically active agent may be administered simultaneously, separately or sequentially.

In certain embodiments, the antibody or antigen-binding fragment thereof, the nucleic acid molecule, the vector, the host cell, the conjugate, the multispecific antibody, or the chimeric antigen receptor or the host cell expressing the chimeric antigen receptor in the pharmaceutical composition of the present invention is sufficient to (e.g., in a subject):
(1) inhibit cell (e.g., tumor cell) proliferation;
(2) inhibit tumor growth;
(3) induce and/or increase antibody-dependent cytotoxic activity;
(4) inhibit HER3-mediated signaling;
(5) prevent and/or treat a HER3-mediated disease/disorder; or
(6) any combination of the above (1) to (5).

In certain embodiments, the HER3-mediated disease/disorder is a tumor, for example a HER3-expressing tumor. In certain embodiments, the tumor is selected from the group consisting of breast cancer, gastric cancer, lung cancer (e.g., non-small cell lung cancer), colorectal cancer, pancreatic cancer, head/neck squamous-cell carcinoma, melanoma, ovarian cancer, prostate cancer, liver cancer, kidney cancer, bladder cancer, or any combination thereof.

Another aspect of the present invention provides a use of the antibody or antigen-binding fragment thereof, the nucleic acid molecule, the vector, the host cell, the conjugate, the multispecific antibody, the chimeric antigen receptor or the host cell expressing the chimeric antigen receptor, or the pharmaceutical composition of the present invention in the manufacture of a medicament, and the medicament is used for: inhibition of cell proliferation, or prevention and/or treatment and/or adjuvant treatment of a tumor.

In certain embodiments, the medicament is used for inhibiting proliferation of a HER3-expressing cell (e.g., tumor cell).

Another aspect of the present invention provides a method for inhibiting cell proliferation, comprising contacting the cell with the antibody or antigen-binding fragment thereof, the nucleic acid molecule, the vector, the host cell, the conjugate, the multispecific antibody, the chimeric antigen receptor or the host cell expressing the chimeric antigen receptor, or the pharmaceutical composition of the present invention. In certain embodiments, the cell is a HER3-expressing cell, such as a tumor cell.

Another aspect of the present invention provides a method for prevention and/or treatment and/or adjuvant treatment of a tumor in a subject, and the method comprises administering to the subject in need thereof an effective amount of the antibody or antigen-binding fragment thereof, the nucleic acid molecule, the vector, the host cell, the conjugate, the multispecific antibody, the chimeric antigen receptor or the host cell expressing the chimeric antigen receptor, or the pharmaceutical composition of the present invention.

In certain embodiments, the method further comprises administering to the subject a second therapy, and the second therapy is selected from the group consisting of surgery, chemotherapy, radiotherapy, immunotherapy, gene therapy, DNA therapy, RNA therapy, nanotherapy, virotherapy, adjuvant therapy, and any combination thereof. In certain embodiments, the second therapy and the methods described above can be administered simultaneously, separately or sequentially.

In any of the above-mentioned embodiments, the tumor related to the antibody or antigen-binding fragment thereof, the nucleic acid molecule, the vector, the host cell, the conjugate, the multispecific antibody, the chimeric antigen receptor or the host cell expressing the chimeric antigen receptor, or the pharmaceutical composition of the present invention can be any tumor type. In certain embodiments, the tumor related to the antibody or antigen-binding fragment thereof, the nucleic acid molecule, the vector, the host cell, the conjugate, the multispecific antibody, the chimeric antigen receptor or the host cell expressing the chimeric antigen receptor, or the pharmaceutical composition of the present invention is a HER3-positive tumor. In certain embodiments, the tumor related to the antibody or antigen-binding fragment thereof, the nucleic acid molecule, the vector, the host cell, the conjugate, the multispecific antibody, the chimeric antigen receptor or the host cell expressing the chimeric antigen receptor, or the pharmaceutical composition of the present invention is selected from the group consisting of breast cancer, gastric cancer, lung cancer (e.g., non-small cell lung cancer), colorectal cancer, pancreatic cancer, head/neck squamous-cell carcinoma, melanoma, ovarian cancer, prostate cancer, liver cancer, kidney cancer, bladder cancer, or any combination thereof.

The antibody or antigen-binding fragment thereof of the present invention and the pharmaceutical composition of the present invention can be formulated into any dosage form known in the medical field, for example, tablet, pill, suspension, emulsion, solution, gel, capsule, powder, granule, elixir, lozenge, suppository, injection (including injectable solution, sterile powder for injection and concentrated solution for injection), inhalant, spray, etc. The preferred dosage form depends on the intended mode of administration and therapeutic use. The pharmaceutical composition of the present invention should be sterile and stable under the conditions of manufacture and storage. A preferred dosage form is injection. Such injection can be a sterile injectable solution. For example, the sterile injectable solution can be prepared by incorporating a necessary dose of the antibody of the present invention into an appropriate solvent, and optionally with other desired ingredients (including, but not limited to, pH adjusting agent, surfactant, adjuvant, ionic strength enhancer, isotonic agent, preservative, diluent, or any combination thereof), and then undergoing filtration and sterilization. In addition, the sterile injectable solution can be prepared as a sterile lyophilized powder (e.g., by vacuum-drying or freeze-drying) for ease of storage and use. Such sterile lyophilized powder can be dispersed in a suitable carrier, such as sterile pyrogen-free water, before use.

Furthermore, the antibody or antigen-binding fragment thereof of the present invention may be presented in a pharmaceutical composition in unit dosage form for ease of administration.

The antibody or antigen-binding fragment thereof and the pharmaceutical composition of the present invention may be administered by any suitable method known in the art, including but not limited to, oral, buccal, sublingual, ophthalmic, topical, parenteral, rectal, intrathecal, intra-cisterna, inguinal, intravesical, local (e.g., powder, ointment, or drop), or nasal route. However, for many therapeutic uses, the preferred route/mode of administration is parenteral administration (e.g., intravenous injection, subcutaneous injection, intraperitoneal injection, intramuscular injection). The skilled artisan will understand that the route and/or mode of administration will vary depending on the intended purpose. In some preferred embodiments, the antibody or antigen-binding fragment thereof, or the pharmaceutical composition of the present invention is administered by intravenous infusion or injection.

The pharmaceutical composition of the present invention may comprise a "therapeutically effective amount" or "prophylactically effective amount" of the antibody or antigen-binding fragment thereof of the present invention. "Prophylactically effective amount" refers to an amount sufficient to prevent, arrest, or delay the onset of a disease. "Therapeutically effective amount" refers to an amount sufficient to cure or at least partially prevent a disease and complications thereof in a patient already suffering from the disease. The therapeutically effective amount of the antibody or antigen-binding fragment thereof of the present invention may vary depending on the severity of the disease to be treated, the general state of the patient's own immune system, the general conditions of the patient such as age, weight and sex, the mode of administration, and other therapy administered at the same time, etc.

In the present invention, the dosing regimen can be adjusted to obtain the optimum desired response (e.g., a therapeutic or prophylactic response). For example, a single dose can be administered; multiple doses can also be administered over a period of time; or the dose can be proportionally reduced or increased according to the level of urgency of the therapeutic situation.

In the present invention, the subject may be a mammal, such as a human.

### Detection application

The antibody or antigen-binding fragment thereof of the present invention can specifically bind to HER3, and thus can be used to detect the presence or level of HER3 in a sample.

Accordingly, in another aspect, the present invention provides a kit, which comprises the antibody or antigen-binding fragment thereof of the present invention. In certain embodiments, the antibody or antigen-binding fragment thereof of the present invention bears a detectable label. In some preferred embodiments, the kit further comprises a second antibody that specifically recognizes the antibody or antigen-binding fragment thereof of the present invention. Preferably, the second antibody further comprises a detectable label.

In the present invention, the detectable label can be any substance that can be detected by fluorescent, spectroscopic, photochemical, biochemical, immunological, electrical, optical or chemical means. It is particularly preferred that such label is suitable for immunological detection (e.g., enzyme-linked immunoassay, radioimmunoassay, fluorescent immunoassay, chemiluminescence immunoassay, etc.). Such labels are well known in the art and include, but are not limited to, enzymes (e.g., horseradish peroxidase, alkaline phosphatase, β-galactosidase, urease, glucose oxidase, etc.), radionuclides (e.g., 3H, 125I, 35S, 14C, or 32P), fluorescent dyes (e.g., fluorescein isothiocyanate (FITC), fluorescein, tetramethylrhodamine isothiocyanate (TRITC), phycoerythrin (PE), Texas Red, rhodamine, quantum dots or cyanine dye derivatives (e.g. Cy7, Alexa 750)), acridinium esters, magnetic beads (e.g. Dynabeads^{®}), calorimetric markers such as colloidal gold or colored glass or plastic (e.g., polystyrene, polypropylene, latex, etc.) beads, and a biotin for binding to an avidin (e.g., streptavidin) modified by the above-mentioned label. In certain embodiments, the detectable label as described above can be attached to the antibody of the present invention via a linker of various lengths to reduce potential steric hindrance.

In another aspect, the present invention provides a method of detecting the presence or level of HER3 in a sample, which comprises a step of using the antibody or antigen-binding fragment thereof of the present invention. In some preferred embodiments, the antibody or antigen-binding fragment thereof of the present invention further bears a detectable label. In some preferred embodiments, the method further comprises detecting the antibody or antigen-binding fragment thereof of the present invention by using a reagent bearing a detectable label. The method can be used for diagnostic purposes, or non-diagnostic purposes (e.g., the sample is a sample of cells rather than a sample from a patient).

In certain embodiments, the method comprises contacting the sample with the antibody or antigen-binding fragment thereof of the present invention under conditions that allow the formation of a complex between the antibody or antigen-binding fragment thereof and HER3, and detecting the formation of the complex.

Given that HER3 is not expressed or is lowly expressed in normal tissues, and is expressed or highly expressed in some cancers, tumors can be diagnosed by detecting the presence or level of HER3 in a sample. Thus, in certain embodiments, the method is used for diagnosing a tumor, such as HER3-positive tumor, such as breast cancer, gastric cancer, lung cancer (e.g., non-small cell lung cancer), colorectal cancer, pancreatic cancer, head/neck squamous-cell carcinoma, melanoma, ovarian cancer, prostate cancer, liver cancer, kidney cancer, bladder cancer, or any combination thereof.

In certain embodiments, the method comprises detecting an expression level of HER3 in a test sample from a subject, and comparing the expression level with a reference value (e.g., a healthy control), wherein an increase of the expression level compared to the reference value is an indication of a tumor.

In another aspect, there is provided a use of the antibody or antigen-binding fragment thereof of the present invention in the manufacture of a kit, in which the kit is used for detecting the presence or level of HER3 in a sample and/or for diagnosing a tumor.

In another aspect, the present invention provides a diagnostic or therapeutic kit, which comprises the antibody or antigen-binding fragment thereof, the nucleic acid molecule, the vector, the host cell, the conjugate, or the multispecific antibody of the present invention, and an instruction for use. The kit may further comprise an administration device for topical administration. The administration device comprises a drug-loading syringe or a needle-free device.

The antibody of the present invention has a high binding affinity to HER3 with an extremely strong specificity, which does not bind to EGFR, HER2 and HER4 of the same family, and can inhibit HER3-mediated signaling and inhibit cell proliferation. Therefore, the antibody of the present invention has the potential to be used for the prevention and/or treatment of a tumor. In addition, the antibody of the present invention is a fully human antibody, which can be safely administered to a human subject without causing an immunogenic response. Therefore, the antibody of the present invention has significant clinical values.

### ABBREVIATION

- CDR: Complementarity determining region in immunoglobulin variable region
- FR: Antibody framework region: amino acid residues in antibody variable region other than CDR residues
- VH: Antibody heavy chain variable region
- VL: Antibody light chain variable region
- IgG: Immunoglobulin G
- IMGT: Numbering system based on The international ImMunoGeneTics information system ^{®} (IMGT) initiated by Lefranc et al., Dev. Comparat. Immunol. 27:55-77, 2003.
- Kabat: Immunoglobulin alignment and numbering system proposed by Elvin A. Kabat (see, for example, Kabat et al., Sequences of Proteins of Immunological Interest, 5th Ed. Public Health Service, National Institutes of Health, Bethesda, Md., 1991).
- Chothia: Immunoglobulin numbering system proposed by Chothia et al., which is a classical rule for identifying the boundaries of CDR regions based on the location of structural loop regions (see, for example, Chothia & Lesk (1987) J. Mol. Biol. 196:901-917; Chothia et al. (1989) Nature 342:878-883).
- mAb: Monoclonal antibody
- EC50: Concentration producing 50% efficacy or binding
- IC50: Concentration producing 50% inhibition
- ELISA: Enzyme-linked immunosorbent assay
- PCR: Polymerase chain reaction
- HRP: Horseradish peroxidase
- K_{D}: Equilibrium dissociation constant
- Ka: Association rate constant
- Kd: Dissociation rate constant
- ADCC: Antibody-dependent cell-mediated cytotoxicity
- FACS: Flow cytometry
- CDR-H1: Complementarity-determining region 1 in immunoglobulin heavy chain variable region
- CDR-H2: Complementarity-determining region 2 in immunoglobulin heavy chain variable region
- CDR-H3: Complementarity-determining region 3 in immunoglobulin heavy chain variable region
- CDR-L1: Complementarity-determining region 1 in immunoglobulin light chain variable region
- CDR-L2: Complementarity-determining region 2 in immunoglobulin light chain variable region
- CDR-L3: Complementarity-determining region 3 in immunoglobulin light chain variable region

### DEFINITIONS

In the present invention, unless otherwise specified, the scientific and technical terms used herein have the meanings commonly understood by those skilled in the art. In addition, the procedures of cell culture, biochemistry, nucleic acid chemistry, and immunology used herein are all routine steps widely used in the corresponding fields. Meanwhile, in order to better understand the present invention, definitions and explanations of related terms are provided below.

As used herein, the term "antibody" is used in the broadest sense and includes various antibody structures, including, but not limited to, monoclonal antibodies, polyclonal antibodies, multispecific antibodies (e.g., bispecific antibodies) and antibody fragments, so long as they exhibit the desired antigen-binding activity. For example, an immunoglobulin molecule may be composed of two pairs of polypeptide chains (each pair having one light chain (LC) and one heavy chain (HC)). Antibody light chains can be classified as κ (kappa) and λ (lambda) light chains. Heavy chains can be classified as µ, δ, γ, α, or ε, and the antibody's isotypes can be defined as IgM, IgD, IgG, IgA and IgE, respectively. Within the light and heavy chains, the variable and constant regions are joined by a "J" region of about 12 or more amino acids, and the heavy chain may further comprise a "D" region of about 3 or more amino acids. Each heavy chain is composed of a heavy chain variable region (VH) and a heavy chain constant region (CH). The heavy chain constant region consists of 3 domains (CH1, CH2 and CH3). Each light chain is composed of a light chain variable region (VL) and a light chain constant region (CL). The light chain constant region consists of one domain, CL. The constant domains are not directly involved in antibody-antigen binding, but exhibit a variety of effector functions, such as mediating the binding of immunoglobulin to host tissues or factors, including various cells (e.g., effector cells) of the immune system and the first component (Clq) of classical complement system. The VH and VL regions can also be subdivided into regions of high variability (called complementarity determining regions (CDRs)) interspersed with more conserved regions called framework regions (FRs). Each VH and VL consists of 3 CDRs and 4 FRs arranged in the following order: FR1, CDR1, FR2, CDR2, FR3, CDR3, FR4, from amino-terminal to carboxy-terminal. The variable regions (VH and VL) of each heavy chain/light chain pair form the antigen binding sites, respectively. The assignment of amino acids in each region or domain can follow the definition of Kabat, Sequences of Proteins of Immunological Interest (National Institutes of Health, Bethesda, Md. (1987 and 1991)), or Chothia & Lesk (1987) J. Mol. Biol. 196 :901-917; Chothia et al. (1989) Nature 342:878-883.

Herein, unless the context clearly dictates otherwise, when the term "antibody" is referred to, it includes not only an intact antibody but also antigen-binding fragments of the antibody.

As used herein, the term "complementarity determining region" or "CDR" refers to the amino acid residues in an antibody variable region that are responsible for antigen binding. The precise boundaries of these amino acid residues can be defined according to various numbering systems known in the art, for example according to the definition by the AbM numbering system (Martin ACR, Cheetham JC, Rees AR (1989) Modeling antibody hypervariable loops: A combined algorithm. Proc Natl Acad Sci USA 86:9268-9272) or the IMGT numbering system (Lefranc et al., Dev. Comparat. Immunol. 27:55-77, 2003). For a given antibody, those skilled in the art will readily identify the CDRs defined by each numbering system. In addition, the correspondence between different numbering systems is well known to those skilled in the art (e.g., see Lefranc et al., Dev. Comparat. Immunol. 27:55-77, 2003).

In the present invention, the CDRs contained in the antibody or antigen-binding fragment thereof of the present invention can be determined according to various numbering systems known in the art. In certain embodiments, the CDRs contained in the antibody or antigen-binding fragment thereof of the present invention are preferably determined by the IMGT, Kabat, Chothia or AbM numbering system.

As used herein, the term "framework region" or "FR" residues refers to those amino acid residues in an antibody variable region other than the CDR residues as defined above.

The term "antibody" is not limited to any particular method for producing the antibody. For example, it comprises recombinant antibody, monoclonal antibody and polyclonal antibody. The antibody can be an antibody of various isotypes, for example an IgG (e.g., IgG1, IgG2, IgG3 or IgG4 subtype), IgAl, IgA2, IgD, IgE or IgM antibody.

As used herein, the term "antigen-binding fragment" of an antibody refers to a molecule other than an intact antibody, which comprises a portion of the intact antibody, and binds to the antigen to which the intact antibody binds. For example, the antigen-binding fragment can be a polypeptide that is a fragment of a full-length antibody, and retains the ability to specifically bind to the same antigen to which the full-length antibody binds, and/or competes with the full-length antibody for specific binding to the antigen, which is also known as an "antigen-binding portion". See, in general, Fundamental Immunology, Ch. 7 (Paul, W., ed., 2nd ed., Raven Press, N.Y. (1989), which is incorporated herein by reference in its entirety for all purposes. An antigen-binding fragment of antibody can be produced by recombinant DNA techniques or by enzymatic or chemical cleavage of an intact antibody. Non-limiting examples of the antigen-binding fragment include Fab, Fab', Fab'-SH, F(ab')₂, Fd, Fv, dAb, and complementary determining region (CDR) fragment, single chain antibody (e.g., scFv), chimeric antibody, diabody, linear antibody, nanobody (technology from Domantis), domain antibody (technology from Ablynx), and a polypeptide comprising at least a part of antibody sufficient to confer the specific antigen-binding ability on the polypeptide. Engineered antibody variants are reviewed by Holliger et al., 2005; Nat Biotechnol, 23: 1126-1136.

As used herein, the term "full-length antibody" refers to an antibody consisting of two "full-length heavy chains" or "heavy chains" and two "full-length light chains" or "light chains", wherein, the "full-length heavy chain" or "heavy chain" refers to such a polypeptide chain, which consists of a heavy chain variable region (VH), a heavy chain constant region CH1 domain, a hinge region (HR), a heavy chain constant region CH2 domain, a heavy chain constant region CH3 domain in the N-terminal to C-terminal direction; and optionally further comprises a heavy chain constant region CH4 domain in the case of IgE isotype. Preferably, the "full-length heavy chain" is a polypeptide chain consisting of VH, CH1, HR, CH2 and CH3 in the N-terminal to C-terminal direction. The "full-length light chain" or "light chain" is a polypeptide chain consisting of a light chain variable region (VL) and a light chain constant region (CL) in the N-terminal to C-terminal direction. The two pairs of full-length antibody chains are linked together by a disulfide bond between CL and CH1 and a disulfide bond between the HRs of the two full-length heavy chains. The full-length antibody of the present invention can be from a single species, such as human; it can also be a chimeric antibody or a humanized antibody. The full-length antibody of the present invention comprises two antigen-binding sites formed by VH and VL pairs respectively, and these two antigen-binding sites specifically recognize/bind to the same antigen.

As used herein, the term "Fd fragment" refers to an antibody fragment consisting of VH and CH1 domains; the term "dAb fragment" refers to an antibody fragment consisting of a VH domain (Ward et al., Nature 341:544 546 (1989)); the term "Fab fragment" refers to an antibody fragment consisting of VL, VH, CL and CH1 domains; the term "F(ab')₂ fragment" refers to an antibody fragment comprising two Fab fragments linked by a disulfide bond on a hinge region; the term "Fab' fragment" refers to a fragment that is obtained after reduction of the disulfide bond linking the two heavy chain fragments in F(ab')₂ fragment, and consists of an intact light chain and an Fd fragment (consisting of VH and CH1 domains) of a heavy chain; the term "Fab'-SH" refers to a Fab fragment containing a free sulfhydryl group.

As used herein, the term "Fv fragment" refers to an antibody fragment consisting of the VL and VH domains of a single arm of an antibody. The Fv fragment is generally considered to be the smallest antibody fragment capable of forming a complete antigen-binding site. It is generally believed that the six CDRs confer antigen-binding specificity to an antibody. However, even a variable region (e.g., Fd fragment, which contains only three CDRs specific for an antigen) is able to recognize and bind to an antigen, although perhaps with a lower affinity than a complete binding site.

As used herein, the term "Fc fragment" refers to an antibody fragment formed by bonding the second and third constant regions of the first heavy chain of an antibody to the second and third constant regions of the second heavy chain via disulfide bonds. The Fc fragment of an antibody has a variety of different functions but are not involved in antigen binding.

As used herein, the term "scFv" refers to a single polypeptide chain comprising VL and VH domains, wherein the VL and VH are linked by a linker (see, for example, Bird et al., Science 242:423 -426 (1988); Huston et al., Proc. Natl. Acad. Sci. USA 85:5879-5883 (1988); and Pluckthun, The Pharmacology of Monoclonal Antibodies, Vol. 113, Roseburg and Moore eds., Springer-Verlag, New York, pp. 269-315 (1994)). Such scFv molecule may have the general structure: NH₂-VL-linker-VH-COOH or NH₂-VH-linker-VL-COOH. Suitable prior art linkers consist of the repeated GGGGS amino acid sequence or variants thereof. For example, a linker having the amino acid sequence (GGGGS)₄ can be used, but variants thereof can also be used (Holliger et al. (1993), Proc. Natl. Acad. Sci. USA 90: 6444-6448). Other linkers useful in the present invention are described by Alfthan et al. (1995), Protein Eng. 8:725-731, Choi et al. (2001), Eur. J. Immunol. 31: 94-106, Hu et al. (1996), Cancer Res. 56:3055-3061, Kipriyanov et al. (1999), J. Mol. Biol. 293:41-56 and Roovers et al. (2001), Cancer Immunol. In some cases, there may also be a disulfide bond between the VH and VL of scFv.

As used herein, the term "diabody" refers to that its VH and VL domains are expressed on a single polypeptide chain, but the linker used is too short to allow pairing between the two domains of the same chain, which forces the domains to pair with the complementary domains of the other chain and create two antigen binding sites (see, for example, Holliger P. et al., Proc. Natl. Acad. Sci. USA 90:6444-6448 (1993), and Poljak R. J. et al., Structure 2:1121-1123 (1994)).

Each of the above antibody fragments maintains the ability to specifically bind to the same antigen to which the full-length antibody binds, and/or competes with the full-length antibody for specific binding to the antigen. Herein, those skilled in the art can obtain an antigen-binding fragment of antibody (for example, the above-mentioned antibody fragment) from a given antibody (for example, the antibody provided by the present invention) using known conventional techniques, and screen the antigen-binding fragment of antibody for specificity in the same manner for the intact antibody.

As used herein, the term "multispecific antibody" refers to an antibody with multiple different antigen-binding specificities, including, for example, bispecific antibody, trispecific antibody, and tetraspecific antibody. "Bispecific antibody" refers to an antibody with two different antigen-binding specificities, which is a conjugate formed by a first antibody (or a fragment thereof) and a second antibody (or a fragment thereof) or an antibody mimetic through a conjugation arm, the conjugation methods include but not limited to chemical reaction, gene fusion and enzymatic catalysis. "Multispecific antibody" includes, for example, trispecific antibody and tetraspecific antibody, in which the trispecific antibody refers to an antibody with three different antigen-binding specificities, and the tetraspecific antibody refers to an antibody with four different antigen-binding specificities.

As used herein, the terms "monoclonal antibody", "McAb", and "mAb" have the same meaning and can be used interchangeably, which refers to an antibody or a fragment of an antibody from a group of highly homogeneous antibody molecules, that is, a population of antibody molecules that are identical except for natural mutations that may arise spontaneously. Monoclonal antibody is highly specific for a single epitope on an antigen. Compared with monoclonal antibody, polyclonal antibody usually contains at least two or more different antibodies, and these different antibodies usually recognize different epitopes on an antigen. In addition, the modifier "monoclonal" only indicates that the antibody is characterized as being obtained from a highly homogeneous antibody population, and cannot be interpreted as requiring any specific method to prepare the antibody.

As used herein, the term "chimeric antibody" refers to an antibody in which part of the light chain or/and heavy chain is derived from an antibody (which may be derived from a specific species or belong to a specific antibody class or subclass), and the other part of the light chain or/and heavy chain is derived from another antibody (which may be derived from the same or different species or belong to the same or different antibody class or subclass), but nevertheless, it still retains the binding activity for the target antigen. For example, the term "chimeric antibody" may include an antibody whose heavy and light chain variable regions are derived from a first antibody (e.g., a human antibody) and whose heavy and light chain constant regions are derived from a second antibody (e.g., a murine antibody). For example, an antibody produced by immunizing fully human transgenic mice may be referred to as a chimeric antibody, which consists of fully human variable regions and murine constant regions.

As used herein, the term "humanized antibody" refers to an antibody that can be prepared by replacing a part of a human antibody with a part of a non-human antibody prepared by immunizing a mammal other than human. Typically, all or part of the CDR regions of a humanized antibody are derived from a non-human antibody (donor antibody), and all or part of the non-CDR regions (e.g., FR regions of variable region and/or constant regions) are derived from a human immunoglobulin (receptor antibody). For example, a humanized antibody can be prepared by grafting CDR sequences derived from the germline of other mammalian species onto human framework sequences.

As used herein, the term "fully human antibody" or "human antibody" refers to an antibody comprising only immunoglobulin sequences derived from human germline. Those skilled in the art understand that a fully human antibody does not exclude the inclusion of amino acid residues not encoded by human germline immunoglobulin sequences, such as mutations introduced by random or in vitro site-specific mutations or by in vivo somatic mutations. Fully human antibodies can be produced by using transgenic mice (XenoMouse (Chemical Biology (2000), vol. 7, issue 8, p. R185-6), HuMAb-Mouse (Infection and Immunity (2002), vol. 70, issue 2, p. 612-9), TC mouse (Biotechnology and Genetics Engineering Reviews (2002), vol. 19, p. 73-82), and KM mouse (Cloning Stem Cells (2002), vol. 4, issue 1, p.91-102)), in which human immunoglobulin genes are transferred, and a large amount of the target antibody can be produced by isolating antibody-producing lymphocytes from mice to form hybridomas. Fully human antibodies can also be produced by phage display (FEBS Letter (1998), vol. 441, p. 20-24). In this method, by using a phage in which a human antibody gene is integrated into a circular single-stranded DNA, fully human antibodies can be expressed on the surface of phage in a form fused to the coat protein of the phage. Fully human antibodies can also be produced by using transgenic mice transferred with human immunoglobulin variable region genes, in which the mice can produce chimeric antibodies consisting of fully human variable regions and murine constant regions, then the fully human variable regions can be linked to human immunoglobulin constant regions using methods known in the art to produce fully human antibodies. For example, a DNA encoding VH can be operably linked to another DNA molecule encoding a heavy chain constant region to obtain a full-length heavy chain gene. The sequence of the human heavy chain constant region gene is known in the art (see, for example, Kabat, E.A. et al. (1991) Sequences of Proteins of Immunological Interest, Fifth Edition, U.S. Department of Health and Human Services, NIH Publication No. 91-3242), and the DNA fragments containing these regions can be obtained by standard PCR amplification. The heavy chain constant region may be an IgGl, IgG2, IgG3, IgG4, IgA, IgE, IgM or IgD constant region, but is generally preferred to be an IgGl or IgG4 constant region. For example, a DNA encoding VL can be operably linked to another DNA molecule encoding a light chain constant region, CL, to obtain a full-length light chain gene (as well as a Fab light chain gene). The sequence of human light chain constant region gene is known in the art (see, for example, Kabat, E.A. et al. (1991) Sequences of Proteins of Immunological Interest, Fifth Edition, U.S. Department of Health and Human Services, NIH Publication No. 91-3242), and the DNA fragments containing these regions can be obtained by standard PCR amplification. The light chain constant region can be a κ or λ constant region, but is generally preferred to be a κ constant region.

As used herein, the term "variant" refers to, in the context of polypeptide (e.g., in the case of polypeptide), a polypeptide or peptide comprising an altered amino acid sequence that has been introduced with a substitution, deletion or addition of amino acid residues. In certain instances, the term "variant" also refers to a polypeptide or peptide that has been modified (i.e., by covalently linking a molecule of any type to the polypeptide or peptide). For example, but not limitation, a polypeptide can be modified by glycosylation, acetylation, pegylation, phosphorylation, amidation, derivatization by known protecting/blocking groups, proteolytic cleavage, attachment to cellular ligands or other proteins, etc. Derivatized polypeptides or peptides can be produced by chemical modification using techniques known to those skilled in the art, including but not limited to specific chemical cleavage, acetylation, formylation, metabolic synthesis of tunicamycin, and the like. Furthermore, a variant has a similar, identical or improved function compared to the polypeptide or peptide from which it is derived.

As used herein, the term "specific binding" refers to a non-random binding reaction between two molecules, such as a reaction between an antibody and an antigen to which it is directed. The strength or affinity of a specific binding interaction can be expressed in term of the equilibrium dissociation constant (KD) or half-maximum effect concentration (EC₅₀) of the interaction.

The specific binding properties between two molecules can be determined using methods known in the art. One method involves measuring the rate of formation and dissociation of antigen binding site/antigen complex. Both the "association rate constant" (ka or kon) and the "dissociation rate constant" (kdis or koff) can be calculated based on the concentration and the actual rate of association and dissociation (see Malmqvist M, Nature, 1993, 361 :186-187). The ratio of kdis/kon is equal to the dissociation constant KD (see Davies et al., Annual Rev Biochem, 1990; 59:439-473). Any effective method can be used to measure KD, kon and kdis values. In certain embodiments, a bioluminescence interferometry method (e.g., ForteBio Octet method) can be used to measure the dissociation constant. In addition, surface plasmon resonance technology (e.g., Biacore) or Kinexa can be used to measure the dissociation constant.

As used herein, the term "vector" refers to a nucleic acid vehicle into which a polynucleotide can be inserted. When a vector enables the expression of a protein encoded by an inserted polynucleotide, the vector is referred to as an expression vector. A vector can be introduced into a host cell by transformation, transduction or transfection, so that the genetic material elements carried by the vector can be expressed in the host cell. Vectors are well known to those skilled in the art and include, but are not limited to: plasmids; phagemids; cosmids; artificial chromosomes, such as yeast artificial chromosomes (YACs), bacterial artificial chromosomes (BACs) or P1-derived artificial chromosomes (PACs); bacteriophages such as λ phage or M13 phage and animal viruses, etc. Animal viruses that can be used as vectors include, but are not limited to, retroviruses (including lentiviruses), adenoviruses, adeno-associated viruses, herpes viruses (e.g., herpes simplex virus), poxviruses, baculoviruses, papillomaviruses, papovavirus (e.g., SV40). A vector may comprise a variety of elements that control expression, including, but not limited to, promoter sequence, transcription initiation sequence, enhancer sequence, selection element, and reporter gene. In addition, the vector may also comprise a replication initiation site.

Expression and cloning vectors contain a nucleic acid sequence that enables the vectors to replicate in one or more selected host cells. Generally, in cloning vectors, this sequence is one that enables the vector to replicate independently of the host chromosomal DNA, and comprises an origin of replication or an autonomously replicating sequence. The term "expression vector" as used herein refers to a vector containing a recombinant polynucleotide, which comprises an expression control sequence operably linked to the nucleotide sequence to be expressed. The expression vector contains sufficient cis-acting elements for expression; other elements for expression can be provided by host cells or in vitro expression systems. Expression vectors include all those known in the art, such as cosmids, plasmids (e.g., naked or contained in liposomes), and viruses (e.g., lentivirus, retrovirus, adenovirus, and adeno-associated virus).

As used herein, the term "host cell" refers to a cell in which a vector can be introduced, which includes, but is not limited to, prokaryotic cells such as *Escherichia coli* or *Bacillus subtilis,* fungal cells such as yeast cell or *Aspergillus,* insect cells such as *S2 Drosophila* cell or Sf9 or animal cells such as fibroblast, NS0 cell, Vero cell, Hela cell, COS cell, CHO cell (e.g., CHO-K1, CHO-S, CHO DXB11, ExpiCHO, CHO DG44 cell), ExpiCHO cell, HEK293 cell, Expi293 cell, BHK cell, and MDCKII cell.

As used herein, the term "identity" refers to the match degree between two polypeptides or between two nucleic acids. When two sequences for comparison have the same monomer sub-unit of base or amino acid at a certain site (e.g., each of two DNA molecules has an adenine at a certain site, or each of two polypeptides has a lysine at a certain site), the two molecules are identical at the site. The percent identity between two sequences is a function of the number of identical sites shared by the two sequences over the total number of sites for comparison × 100. For example, if 6 of 10 sites of two sequences are matched, these two sequences have an identity of 60%. For example, DNA sequences: CTGACT and CAGGTT share an identity of 50% (3 of 6 sites are matched). Generally, the comparison of two sequences is conducted in a manner to produce maximum identity. Such alignment can be conducted by using a computer program such as Align program (DNAstar, Inc.) which is based on the method of Needleman, et al. (J. Mol. Biol. 48:443-453, 1970). The percent identity between two amino acid sequences can also be determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percentage of identity between two amino acid sequences can be determined by the algorithm of Needleman and Wunsch (J. Mol. Biol. 48:444-453 (1970)) which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6.

As used herein, the term "conservative substitution" refers to an amino acid substitution that does not adversely affect or alter the expected properties of protein/polypeptide comprising the amino acid sequence. For example, a conservative substitution may be introduced by a standard technique known in the art, such as site-directed mutagenesis and PCR-mediated mutagenesis. Conservative amino acid substitutions include substitutions of amino acid residues with amino acid residues having similar side chains, for example, substitutions with amino acid residues that are physically or functionally similar (e.g., have similar size, shape, charge, chemical properties, including ability to form covalent or hydrogen bonds, etc.) to the corresponding amino acid residues. Families of amino acid residues with similar side chains has been defined in the art. These families include amino acids with basic side chains (e.g., lysine, arginine, and histidine), acidic side chains (e.g., aspartic acid, glutamic acid), uncharged polar side chains (e.g., glycine, asparagine, glutamine, serine, threonine, tyrosine, cysteine, tryptophan), non-polar side chains (e.g. alanine, valine, leucine, isoleucine, proline, phenylalanine, methionine), β-branched side chains (e.g., threonine, valine, isoleucine) and aromatic side chains (e.g., tyrosine, phenylalanine, tryptophan, histidine). Therefore, it is preferred to replace the corresponding amino acid residue with another amino acid residue from the same side chain family. Methods for identifying conservative substitutions of amino acids are well known in the art (see, for example, Brummell et al., Biochem. 32: 1180-1187 (1993); Kobayashi et al. Protein Eng. 12 (10): 879-884 (1999); and Burks et al. Proc. Natl Acad. Set USA 94: 412-417 (1997), which are incorporated herein by reference).

The twenty conventional amino acids involved in herein have been written follows conventional usage. See, for example, Immunology-A Synthesis (2nd Edition, E. S. Golub and D. R. Gren, Eds., Sinauer Associates, Sunderland, Mass. (1991)), which is incorporated herein by reference. In the present invention, the terms "polypeptide" and "protein" have the same meaning and are used interchangeably. And in the present invention, amino acids are generally represented by single-letter and three-letter abbreviations known in the art. For example, alanine can be represented by A or Ala.

As used herein, the term "pharmaceutically acceptable carrier and/or excipient" refers to a carrier and/or excipient that is pharmacologically and/or physiologically compatible with a subject and an active ingredient, which is well known in the art (see, for example, Remington's Pharmaceutical Sciences. Edited by Gennaro AR, 19th ed. Pennsylvania: Mack Publishing Company, 1995) and includes, but is not limited to: pH adjuster, surfactant, adjuvant, ionic strength enhancer, diluent, agent for maintaining osmotic pressure, agent for delaying absorption, preservative. For example, the pH adjusting agent includes, but is not limited to, phosphate buffered saline. The surfactant includes, but is not limited to, cationic, anionic or non-ionic surfactant, such as Tween-80. The ionic strength enhancer includes, but is not limited to, sodium chloride. The preservative includes, but is not limited to, various antibacterial and antifungal agents, such as paraben, trichloro-t-butanol, phenol, sorbic acid, and the like. The agent for maintaining osmotic pressure includes, but is not limited to, sugar, NaCl, and the like. The agent for delaying absorption includes, but is not limited to, monostearate and gelatin. The diluent includes, but is not limited to, water, aqueous buffer (e.g., buffered saline), alcohol and polyol (e.g., glycerol), and the like. The preservative includes, but is not limited to, various antibacterial and antifungal agents, such as thimerosal, 2-phenoxyethanol, paraben, trichloro-t-butanol, phenol, sorbic acid, and the like. The stabilizer has the meaning commonly understood by those skilled in the art, which can stabilize the desired activity of active ingredient in drug, including but not limited to sodium glutamate, gelatin, SPGA, saccharide (e.g., sorbitol, mannitol, starch, sucrose, lactose, dextran, or glucose), amino acid (e.g., glutamic acid, glycine), protein (e.g., dried whey, albumin, or casein) or degradation product thereof (e.g., lactalbumin hydrolysate), etc.

As used herein, the term "prevention" refers to a method performed to prevent or delay the occurrence of a disease or disorder or symptom (e.g., a tumor) in a subject. As used herein, the term "treatment" refers to a method performed to obtain a beneficial or desired clinical result. For the purposes of the present invention, a beneficial or desired clinical outcome includes, but is not limited to, alleviation of symptom, reduction in the extent of disease, stabilization (i.e., not worsening) of disease state, delaying or slowing the progression of disease, amelioration or remission of disease state, and relief of symptom (whether in part or in whole), whether detectable or undetectable. Additionally, "treatment" can also refer to a prolonging survival as compared to the expected survival if not receiving treatment.

As used herein, the term "subject" refers to a mammal, such as a primate mammal, such as a human. In certain embodiments, the subject (e.g., human) has a tumor, or is at risk of having a disease as described above.

As used herein, the term "effective amount" refers to an amount sufficient to obtain or at least partially obtain a desired effect. For example, an effective amount for preventing a disease (e.g., tumor) refers to an amount sufficient to prevent, arrest or delay the occurrence of disease (e.g., tumor); an effective amount for treating a disease refers to an amount sufficient to cure or at least partially prevent a disease and complications thereof in a patient who has already suffered from the disease. It is completely within the ability of those skilled in the art to determine such an effective amount. For example, the effective amount for a therapeutic use will depend on the severity of the disease to be treated, the overall state of the patient's own immune system, the patient's general conditions such as age, weight and gender, the mode of administration, and other therapies administered simultaneously.

As used herein, the term "effector function" refers to a biological activity that is attributable to an antibody Fc region (a Fc region with natural sequence or a Fc region with amino acid sequence variant), and which varies with the isotype of antibody. Examples of antibody effector function include but are not limited to: Fc receptor binding affinity, antibody-dependent cell-mediated cytotoxicity (ADCC), complement-dependent cytotoxicity (CDC), antibody-dependent cellular phagocytosis (ADCP), down-regulation of cell surface receptor (e.g., B cell receptor), B cell activation, cytokine secretion, half-life/clearance rate of antibody and antigen-antibody complex, etc. Methods of changing the effector function of antibody are known in the art, for example, by introducing a mutation into the Fc region.

As used herein, the term "antibody-dependent cell-mediated cytotoxicity (ADCC)" refers to a form of cytotoxicity in which cytotoxic effector cells specifically bind to the target cells to which the antigen is attached, through the binding of Ig to an Fc receptor (FcR) presented on cytotoxic cells (e.g., natural killer (NK) cells, neutrophils or macrophages), and then kills the target cells by secreting cytotoxins.

As used herein, the term "antibody-mediated internalization" refers to a phenomenon that an antibody crosses a cell membrane after binding to a cell surface antigen. The internalization includes antibody-mediated internalization of a receptor (e.g., HER3).

Herein, the combination therapy comprises using a combination of an anti-HER3 antibody or antigen-binding fragment thereof encompassed by the present invention with one or more additional active therapeutic agents (e.g., chemotherapeutic agents) of a second therapy or other modes of prophylaxis or treatment (e.g., radiation therapy).

In such combination therapy, the various active agents often have different complementary mechanisms of action, and the combination therapy may result in a synergistic effect. The combination therapy comprises a therapeutic agent that affects an immune response (e.g., enhancing or activating the response) and a therapeutic agent that affects (e.g., inhibits or kills) a tumor/cancer cell. The combination therapy can reduce the likelihood of occurrence of drug-resistant cancer cells. The combination therapy may allow dose reduction of one or more agents to reduce or eliminate adverse effects associated with the one or more of the agents. Such combination therapy may have a synergistic therapeutic or prophylactic effect on the potential disease, disorder or condition.

Herein, the "combination" comprises therapies that may be administered separately, for example, therapies can be formulated separately (e.g., can be provided in a kit) for separate administration, or can be administered together in a single formulation (i.e., "co-formulation"). In certain embodiments, the anti-HER3 antibodies or antigen-binding fragments thereof of the present invention may be administered sequentially. In other embodiments, the anti-HER3 antibodies or antigen-binding fragments thereof may be administered concurrently. The anti-antibodies or antigen-binding fragments thereof of the present invention may be used in any combination with at least one additional (active) agent.

In this context, the HER3-positive is obtained by immunohistochemistry and evaluation of staining intensity by professional clinical pathologists.

The terms "cancer" and "tumor" are used interchangeably and refer to a broad group of diseases characterized by the uncontrolled growth of abnormal cells in the body. Unregulated cell division may lead to the formation of malignant tumors or cells that invade adjacent tissues and may metastasize to distant sites in the body through the lymphatic system or bloodstream. The cancer includes a benign or malignant cancer, as well as a dormant tumor or micrometastasis. The cancer also includes hematological malignancies.

The term "hematological malignancy" includes lymphoma, leukemia, myeloma or lymphoid malignancy, as well as spleen cancer and lymph node tumor. Exemplary lymphoma includes B-cell lymphoma and T-cell lymphoma. B-cell lymphoma includes, for example, Hodgkin's lymphoma. T-cell lymphoma includes, for example, cutaneous T-cell lymphoma. Hematological malignancy also includes leukemia, such as secondary leukemia or acute lymphocytic leukemia. Hematological malignancy also includes myeloma (e.g., multiple myeloma) and other hematological and/or B- or T-cell-related cancers.

As used herein, the term "about" or "approximately" when applied to a numerical variable generally means that the variable has a value within experimental error (e.g., within a 95% confidence interval for the mean) or within ± 10% or wider range of a specified value.

Embodiments of the present invention will be described in detail below with reference to the drawings and examples, but those skilled in the art will understand that the following drawings and examples are only for illustrating the present invention, rather than limiting the scope of the present invention. Various objects and advantages of the present invention will become applicable to those skilled in the art from the accompanying drawings and the following detailed description of preferred embodiments.

### BRIEF DESCRIPTION OF THE DRAWINGS

FIG. 1A shows the determination of the binding activity of anti-human HER3 fully human antibody to human HER3 protein.
FIG. 1B shows the determination of the binding activity of anti-human HER3 fully human antibody to monkey HER3 protein.
FIG. 1C shows the determination of the binding activity of anti-human HER3 fully human antibody to rat HER3 protein.
FIG. 2A shows the determination of the binding activity of anti-human HER3 fully human antibody to human breast cancer cell T47D.
FIG. 2B shows the determination of the binding activity of anti-human HER3 fully human antibody to human gastric cancer cell NCI-N87.
FIG. 2C shows the determination of the binding activity of anti-human HER3 fully human antibody to human non-small cell lung cancer cell NCI-H358.
FIG. 3 shows the detection of the ADCC activity of anti-human HER3 fully human antibody.
FIG. 4 shows the detection of the inhibition of AKT phosphorylation by anti-human HER3 fully human antibody.
FIG. 5A shows the detection of internalization activity of anti-human HER3 fully human antibody on human breast cancer cell MDA-MB-453.
FIG. 5B shows the detection of internalization activity of anti-human HER3 fully human antibody on human colorectal cancer cell WIDR.
FIG. 5C shows the detection of internalization activity of anti-human HER3 fully human antibody on human non-small cell lung cancer cell NCI-H358.
FIG. 5D shows the detection of internalization activity of anti-human HER3 fully human antibody on human pancreatic cancer cell HPAF-II.
FIG. 6 shows the detection of binding domain of anti-human HER3 fully human antibody.
FIG. 7A shows the competitive binding assay of ELISA between 100H7D3-hIgG, 47A3E3-hIgG and control antibody U1-59.
FIG. 7B shows the competitive binding assay of ELISA between 47A3E3-hIgG, 100H7D3-hIgG and 22B6D2-hIgG.
FIG. 7C shows the competitive binding assay of ELISA between 100H7D3-hIgG and 47A3E3-hIgG.
FIG. 8 shows the competitive binding assay of FACS detection between 22B6D2-hIgG, 47A3E3-hIgG, 100H7D3-hIgG and control antibody U1-59.
FIG. 9 shows the detection of binding specificity of anti-human HER3 fully human antibody.

### SEQUENCE INFORMATION

Information of sequences involved in the present invention is described in Table 1 below:

**Table 1: Information of sequences involved in the present invention**

| SEQ ID NO: | Description | SEQ ID NO: | Description |
|---|---|---|---|
| 1 | 22B6D2 VH amino acid sequence | 37 | 47A3E3 VH CDR1 (IMGT definition) |
| 2 | 22B6D2 VL amino acid sequence | 38 | 47A3E3 VH CDR2 (IMGT definition) |
| 3 | 44A4A11 VH amino acid sequence | 39 | 47A3E3 VH CDR3 (IMGT definition) |
| 4 | 44A4A11 VL amino acid sequence | 40 | 47A3E3 VH CDR1 (Kabat definition) |
| 5 | 47A3E3 VH amino acid sequence | 41 | 47A3E3 VH CDR2 (Kabat definition) |
| 6 | 47A3E3 VL amino acid sequence | 42 | 47A3E3 VH CDR3 (Kabat/Chothia definition) |
| 7 | 100H7D3 VH amino acid sequence | 43 | 47A3E3 VH CDR1 (Chothia definition) |
| 8 | 100H7D3 VL amino acid sequence | 44 | 47A3E3 VH CDR2 (Chothia definition) |
| 9 | 22B6D2 VH CDR1 (IMGT definition) | 45 | 100H7D3 VH CDR1 (IMGT definition) |
| 10 | 22B6D2 VH CDR2 (IMGT definition) | 46 | 100H7D3 VH CDR2 (IMGT definition) |
| 11 | 22B6D2 VH CDR3 (IMGT definition) | 47 | 100H7D3 VH CDR3 (IMGT definition) |
| 12 | 22B6D2 VH CDR1 (Kabat definition) | 48 | 100H7D3 VH CDR1 (Kabat definition) |
| 13 | 22B6D2 VH CDR2 (Kabat definition) | 49 | 100H7D3 VH CDR2 (Kabat definition) |
| 14 | 22B6D2 VH CDR3 (Kabat definition) | 50 | 100H7D3 VH CDR3 (Kabat/Chothia definition) |
| 15 | 22B6D2 VH CDR1 (Chothia definition) | 51 | 100H7D3 VH CDR1 (Chothia definition) |
| 16 | 22B6D2 VH CDR2 (Chothia definition) | 52 | 100H7D3 VH CDR2 (Chothia definition) |
| 17 | 22B6D2 VH CDR3 (Chothia definition) | 53 | 100H7D3 VL CDR1 (IMGT definition) |
| 18 | 22B6D2 VL CDR1 (IMGT definition) | 54 | 100H7D3 VL CDR3 (IMGT/Kabat/Chothia definition) |
| 19 | 22B6D2 VL CDR2 (IMGT definition) | 55 | 22B6D2 VH nucleotide sequence |
| 20 | 22B6D2 VL CDR3 (IMGT definition) | 56 | 22B6D2 VL nucleotide sequence |
| 21 | 22B6D2 VL CDR1 (Kabat definition/Chothia definition) | 57 | 44A4A11 VH nucleotide sequence |
| 22 | 22B6D2 VL CDR2 (Kabat definition/Chothia definition) | 58 | 44A4A11 VL nucleotide sequence |
| 23 | 22B6D2 VL CDR3 (Kabat/Chothia definition) | 59 | 47A3E3 VH nucleotide sequence |
| 24 | 44A4A11 VH CDR1 (IMGT definition) | 60 | 47A3E3 VL nucleotide sequence |
| 25 | 44A4A11 VH CDR2 (IMGT definition) | 61 | 100H7D3 VH nucleotide sequence |
| 26 | 44A4A11 VH CDR3 (IMGT definition) | 62 | 100H7D3 VL nucleotide sequence |
| 27 | 44A4A11 VH CDR1 (Kabat definition) | 63 | IgG1 heavy chain constant region amino acid sequence |
| 28 | 44A4A11 VH CDR2 (Kabat definition) | 64 | IgG1 heavy chain constant region nucleotide sequence |
| 29 | 44A4A11 VH CDR3 (Kabat/Chothia definition)) | 65 | Light chain constant region amino acid sequence |
| 30 | 44A4A11 VH CDR1 (Chothia definition) | 66 | Light chain constant region nucleotide sequence |
| 31 | 44A4A11 VH CDR2 (Chothia definition) | 67 | 44A4A11 VL CDR1 (IMGT definition) |
| 32 | 47A3E3 VL CDR1 (IMGT definition) | 68 | 44A4A11 VL CDR2 (IMGT definition) |
| 33 | 47A3E3/100H7D3 VL CDR2 (IMGT definition) | 69 | 44A4A11 VL CDR3 (IMGT/Kabat/Chothia definition) |
| 34 | 47A3E3 VL CDR3 (IMGT/Kabat/Chothia definition) | 70 | 44A4A11 VL CDR1 (Kabat/Chothia definition) |
| 35 | 47A3E3 VL CDR1 (Kabat/Chothia definition) | 71 | 44A4A11 VL CDR2 (Kabat/Chothia definition) |
| 36 | 47A3E3/100H7D3 VL CDR2 (Kabat/Chothia definition) | 72 | 100H7D3 VL CDR1 (Kabat/Chothia definition) |

### EXAMPLES

The present invention will now be described with reference to the following examples, which are intended to illustrate the present invention, but not to limit it.

Unless otherwise specified, the molecular biology experiment methods and immunoassay methods used in the present invention are performed by basically referring to the methods described by J. Sambrook et al., Molecular Cloning: A Laboratory Manual, 2nd Edition, Cold Spring Harbor Laboratory Press, 1989, and F. M. Ausubel et al., Short Protocols in Molecular Biology, 3rd Edition, John Wiley & Sons, Inc., 1995. Those skilled in the art understand that the examples describe the present invention by way of example and are not intended to limit the scope of the claimed invention.

### Example 1: Preparation of HER3 antigen and control antibody

### 1.1 Preparation of HER3 antigen

Extracellular sequences of human HER3 (UniProt: P21860) and monkey HER3 (UniProt: G7N7B1) were synthesized by GenScript and constructed on pTT5 vector, with six histidines introduced at the C-terminal; recombinant plasmids were extracted, and then transiently transfected into HEK293 cells to perform transient expression after being verified by sequencing; supernatants were collected on Day 6, and purified to obtain human HER3 protein and monkey HER3 protein, respectively.

### 1.2 Expression of HER3 control antibody

The HER3 control antibody was derived from U1-59 of the patent CN200680049887. The nucleotide sequences of the heavy and light chains of the antibody after codon optimization by GenScript were synthesized and cloned into pTT5 vector respectively, and then the pcDNA3.4 plasmids corresponding to the nucleotides of the heavy and light chains of the antibody were simultaneously transfected into CHOS cells (Thermo). After the cell supernatant was collected by centrifugation, the supernatant was purified with Protein A (MabSelect SuRe, GE) to obtain the control antibody.

### Example 2: Preparation of anti-human HER3 monoclonal antibody

### 2.1 Immunization of mice

The fully human transgenic mouse H2L2 (the antibody produced by this mouse was a chimeric antibody composed of fully human variable regions and mouse constant regions) from Harbor BioMed was used, and immunized with human HER3 protein prepared in 1.1. Freund's complete adjuvant, Adjuvant system (Sigma), were used for immunization by foot pad, subcutaneous, tail root, or intraperitoneal injection, or Titermax Gold Adjuvant (Sigma), Imject Alum (Thermo) were used to perform immunization by foot pad injection once every 2-3 days. During the immunization period, the serum titer of anti-HER3 antibodies was monitored every two weeks by ELISA method. Further, the protocols 2.2 and 2.3 below were used to generate mouse hybridomas with optimal titers.

### 2.2 Fusion and screening of hybridoma

1 µg/mL human HER3 protein was coated on a ELISA plate (BIOFIL) overnight at 4°C, with 100 µL/well; after washing once with 300 µL of PBST (0.2% Tween-20), 100 µl of 2% BSA in PBS was added to each well, and incubated at 37°C for 1 hour; and 20 µl of the hybridoma supernatant was directly added to the ELISA plate, and incubated at 37°C for 2 hours. After discarding the solution, the ELISA plate was machine-washed 3 times with PBST (0.2% Tween-20), 320 µl per well. The ELISA plate was dried then added with 100 µL of (1:5000) diluted HRP conjugated Goat anti-Rat IgG (Thermo Fisher) to each well, and incubated at 37°C for 1 hour. The solution was discarded, and the ELISA plate was machine-washed 5 times with PBST (0.2% Tween-20), 320 µl per well. The ELISA plate was dried then added with 100 µL of TMB (InnoReagents) to each well for chromogenic reaction in the dark, then added with 50 µL of 2N H₂SO₄ to stop the chromogenic reaction, and read for the absorbance at 450 nm with a microplate reader. The clones with an OD450 nm value greater than 5 times that of the negative control were identified as positive clones. The positive clones were then subjected to ELISA for the cross-reactivity on monkey HER3 protein (referring to 2.1), and cell binding detection with human breast cancer T47D cells, and the optimal clones were selected for subcloning. Subclones were screened by similar method, and the optimal subclones were selected to purify chimeric antibodies.

### Example 3: Evaluation of anti-human HER3 chimeric antibody

### 3.1 Evaluation of binding activity of anti-human HER3 chimeric antibody

All the preferred monoclones were transferred to 6-well plate for serum-free culture, and then 4~6 ml of culture supernatant was subjected to affinity purification with Protein-A beads; the antibody proteins bound to Protein-A beads were eluted with 1M Glycine solution (pH 2.5); the antibody proteins after elution were neutralized with 1M Tris solution and the antibody proteins were quantified by Nanodrop for concentration and subjected to candidate evaluation.

ELISA affinity determination of HER3 chimeric antibody: Human and monkey HER3 proteins were diluted to 1 µg/ml with CBS coating solution, added to ELISA plate with 100 ng/well, and allowed to stand overnight at 4°C; the ELISA plate was washed once with PBS on the next day; then 100 µl of 2% BSA was added to each well, and incubated at 37°C for 2 hours; the purified chimeric antibody and control antibody were serially diluted with 2% BSA (starting from 10 µg/ml, with 3-fold gradient, 8 concentration points), respectively; the blocking solution was removed, then the diluted antibody solution was added to the ELISA plate, and incubated at 37°C for 2 hours; the plate was washed 3 times with PBST (0.2% Tween-20), 320 µl per well; after the plate was dried, 100 µl of HRP conjugated Goat anti-Rat IgG (Thermo) was added to each well, and incubated at 37°C for 1 hour. The plate was washed 5 times with PBST (0.2% Tween-20). After the plate was dried, 100 µl of TMB (CoWin Biotech) chromogenic substrate was added to each well, reacted at room temperature for 3-5 minutes; 50 µl of 2N H₂SO₄ was added to each well to stop the chromogenic reaction, and absorbance value at OD450 nm was read with a microplate reader. The raw data were imported into GraphPad Prism 6 software for nonlinear curve fitting, and the EC50 values of each antibody binding to human HER3 and monkey HER3 were calculated. As shown in Table 2 below, all the screened antibodies could bind to human and monkey HER3 proteins.

**Table 2: Detection of chimeric antibody binding to HER3 protein**

| Antibody | Human HER3 EC50, ng/mL | Monkey HER3 EC50, ng/mL |
|---|---|---|
| 44A4A11 | 12.1 | 6.5 |
| 100H7D3 | 13.1 | 18.7 |
| 22B6D2 | 5.4 | 7.1 |
| 47A3E3 | 9.6 | 10.1 |

### 3.2 Acquisition of sequences of anti-human HER3 chimeric antibody

The hybridoma cells were cultured to reach about 8000 cells, and then the cells were lysed; and the first-strand cDNA was synthesized using a cDNA reverse transcription kit (Thermo Fisher). VH and VK genes were amplified from cDNA by PCR using primers, and the PCR products were purified by DNA purification kit (Qiagen), and ligated to TOPO vector (Thermo Fisher Sci). About 12 clones were picked in each ligation reaction for sequencing. The sequences were analyzed by abYsis. The obtained variable region sequences and CDR sequences of anti-human HER3 antibodies were shown in Table 3-1 and Table 3-2.

**Table 3-1: CDR sequences of anti-human HER3 antibodies**

| Antibody | Definition | Heavy chain CDR1 | Heavy chain CDR2 | Heavy chain CDR3 | Light chain CDR1 | Light chain CDR2 | Light chain CDR3 |
|---|---|---|---|---|---|---|---|
| 22B6D2 | IMGT | GFTFRSYG (SEQ ID NO: 9) | IWYDGSNK (SEQ ID NO:10) | ARYYYDSN GYYDΛFDI (SEQ ID NO:11) | QGISNY (SEQ ID NO:18) | AAS (SEQ ID NO:19) | QQLNSYPIT (SEQ ID NO:20) |
| | Kabat | SYGMH (SEQ ID NO:12) | VIWYDGSN KYYADSVK G (SEQ ID NO:13) | YYYDSNGY YDAFDI (SEQ ID NO:14) | RASQGISN YLA (SEQ ID NO:21) | AASTLQS (SEQ ID NO:22) | QQLNSYPIT (SEQ ID NO:23) |
| | Chothia | GFTFRSY (SEQ ID NO:15) | WYDGSN (SEQ ID NO:16) | YYYDSNGY YDAFDI (SEQ ID NO:17) | RASQGISN YLA (SEQ ID NO:21) | AASTLQS (SEQ ID NO:22) | QQLNSYPIT (SEQ ID NO:23) |
| 44A4A11 | IMGT | GFTFSSYA (SEQ ID NO:24) | ISDSGGST (SEQ ID NO:25) | AKDGGISM VRGVIIEEG YFDL (SEQ ID NO:26) | QGISSY (SEQ ID NO:67) | VVS (SEQ ID NO:68) | HQLNSYPL T (SEQ ID NO:69) |
| | Kabat | SYAMN (SEQ ID NO:27) | SISDSGGST YYADSVKG (SEQ ID NO:28) | DGGISMVR GVIIEEGYF DL (SEQ ID NO:29) | RASQGISSY LA (SEQ ID NO:70) | VVSTLQS (SEQ ID NO:71) | HQLNSYPL T (SEQ ID NO:69) |
| | Chothia | GFTFSSY (SEQ ID NO:30) | SDSGGS (SEQ ID NO:31) | DGGISMVR GVIIEEGYF DL (SEQ ID NO:29) | RASQGISSY LA (SEQ ID NO:70) | VVSTLQS (SEQ ID NO:71) | HQLNSYPL T (SEQ ID NO:69) |
| 47A3E3 | IMGT | GDSVSSSQS T (SEQ ID NO:37) | TFYRSRWY N (SEQ ID NO:38) | ARDTYYYG SGGFDY (SEQ ID NO:39) | QSLSRW (SEQ ID NO:32) | KAS (SEQ ID NO:33) | QQYKNYYS (SEQ ID NO:34) |
| | Kabat | SSQSTWN (SEQ ID NO:40) | RTFYRSRW YNDYALSM KS (SEQ ID NO:41) | DTYYYGSG GFDY (SEQ ID NO:42) | RASQSLSR WLA (SEQ ID NO:35) | KASSLES (SEQ ID NO:36) | QQYKNYYS (SEQ ID NO:34) |
| | Chothia | GDSVSSSQS (SEQ ID NO:43) | FYRSRWY (SEQ ID NO:44) | DTYYYGSG GFDY (SEQ ID NO:42) | RASQSLSR WLA (SEQ ID NO:35) | KASSLES (SEQ ID NO:36) | QQYKNYYS (SEQ ID NO:34) |
| 100H7D3 | IMGT | GFTFSRNA (SEQ ID NO:45) | IWHDGSNK (SEQ ID NO:46) | ARDRGASD F (SEQ ID NO:47) | QSINNW (SEQ ID NO:53) | KAS (SEQ ID NO:33) | QQYNSYSL T (SEQ ID NO:54) |
| | Kabat | RNAMH (SEQ ID NO:48) | VIWHDGSN KYYGDSVK G (SEQ ID NO:49) | DRGASDF (SEQ ID NO:50) | RASQSINN WLA (SEQ ID NO:72) | KASSLES (SEQ ID NO:36) | QQYNSYSL T (SEQ ID NO:54) |
| | Chothia | GFTFSRN (SEQ ID NO:51) | WHDGSN (SEQ ID NO:52) | DRGASDF (SEQ ID NO:50) | RASQSINN WLA (SEQ ID NO:72) | KASSLES (SEQ ID NO:36) | QQYNSYSL T (SEQ ID NO:54) |

**Table 3-2: Variable region sequences of anti-human HER3 antibodies**

| Antibody | Heavy chain variable region (SEQ ID NO:) | Light chain variable region (SEQ ID NO:) |
|---|---|---|
| 22B6D2 | 1 | 2 |
| 44A4A11 | 3 | 4 |
| 47A3E3 | 5 | 6 |
| 100H7D3 | 7 | 8 |

### Example 4: Evaluation of anti-human HER3 fully human antibody

### 4.1 Construction of anti-human HER3 fully human antibody

The amino acid sequences of the light chain variable regions of 22B6D2, 47A3E3 and 100H7D3 were ligated to the amino acid sequence (SEQ ID NO: 65) of the human light chain κ constant region, respectively, and the amino acid sequences of the heavy chain variable regions thereof were ligated to the amino acid sequence (SEQ ID NO: 63) of the human IgG1 heavy chain constant region, respectively, to construct HER3 fully human antibodies, 22B6D2-hIgG, 47A3E3-hIgG, and 100H7D3-hIgG. The cDNA synthesized by codon optimization was ligated into plasmid pTT5 (by GenScript). The pTT5 plasmids corresponding to the heavy chain and light chain nucleotides of the fully human antibody were simultaneously transfected into CHO-S cells, the supernatant was collected by centrifugation, and the recombinant antibody in the supernatant was purified using Protein A (MabSelect SuRe, GE) to obtain the anti-human HER3 fully human antibody.

### 4.2 Detection of binding activity of anti-human HER3 fully human antibody

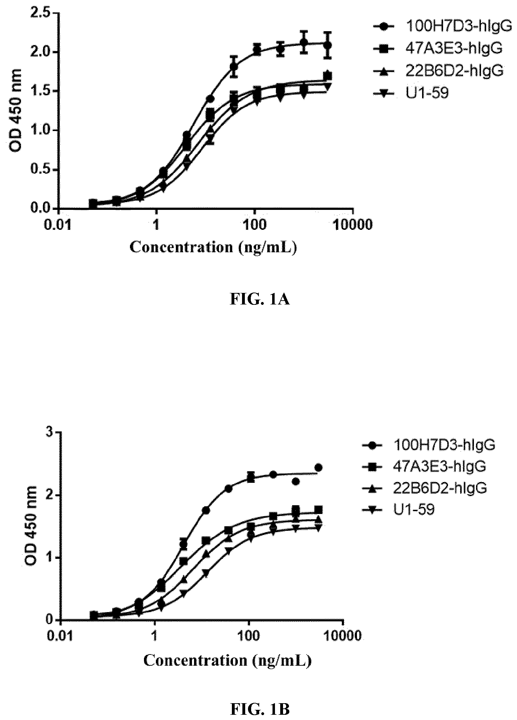

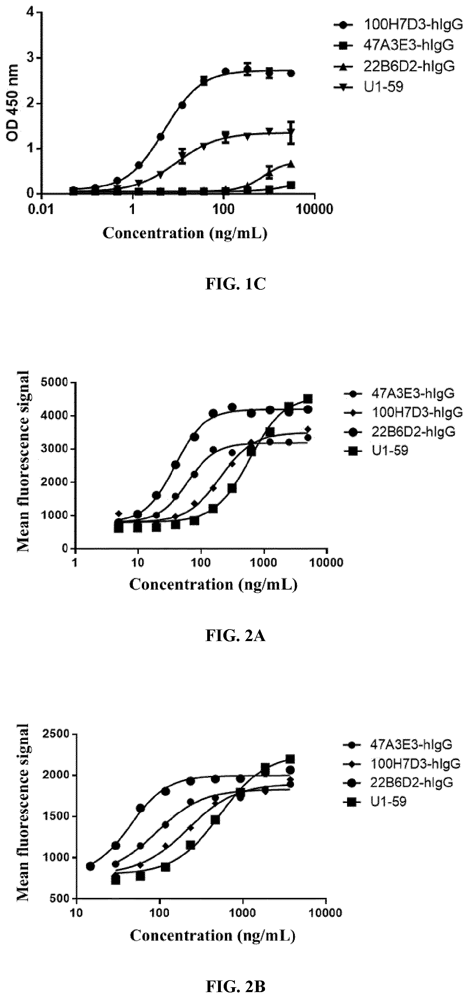

To detect the affinity of the anti-human HER3 fully human antibody to human, monkey, and rat HER3 proteins (UniProt: Q62799), the human, monkey and rat HER3 proteins were diluted to 1 µg/ml with CBS coating solution, coated on a 96-well ELISA plate, with 100 µl/well, and allowed to stand overnight at 4°C; the ELISA plate was washed once with PBS, dried, then 100 µl of 2% BSA was added to each well, incubated at 37°C for 2 hours, and then the blocking solution was removed; the human antibody and control antibody were serially diluted with 2% BSA (starting from 5µg/ml, with 3-fold gradient, 11 concentration points), added to the ELISA plate, with 100µl per well, and incubated at 37°C for 2 hours; the plate was washed with PBST (0.2% Tween-20) 3 times, and dried, then 100 µl of HRP Goat Anti-Human IgG (H+L) (Jackson) was added to each well, and incubated at 37°C for 1 hour. The plate was washed 5 times with PBST (0.2% Tween-20), and dried, then 100 µl of TMB (InnoReagents) chromogenic substrate was added to each well, reacted at room temperature for 3-5 minutes, then 50 µl of 2N H₂SO₄ was added to each well to stop the chromogenic reaction, and the absorbance value at OD450 nm was read with a microplate reader. The raw data were imported into GraphPad Prism 6 software for nonlinear curve fitting, and EC50 values were calculated. The results were shown in FIG. 1A, FIG. 1B, FIG. 1C and Table 4. 22B6D2-hIgG, 47A3E3-hIgG, 100H7D3-hIgG all had good binding affinity to human and monkey proteins; and 100H7D3-hIgG had good binding to rat protein, while 22B6D2- hIgG, 47A3E3-hIgG basically did not bind to rat protein.

**Table 4: Results of binding of anti-human HER3 fully human antibodies to human, monkey and rat HER3-His proteins**

| Antibody | Human HER3-His EC50 (ng/mL) | Monkey HER3-His EC50 (ng/mL) | Rat HER3-His EC50 (ng/mL) |
|---|---|---|---|
| U1-59 | 5.756 | 4.194 | 4.992 |
| 22B6D2-hIgG | 4.065 | 3.629 | Not binding |
| 47A3E3-hIgG | 7.127 | 6.657 | Not binding |
| 100H7D3-hIgG | 8.491 | 13.08 | 9.37 |

### 4.3 Detection of dynamic affinity of anti-human HER3 fully human antibody

The dynamic affinity of candidate antibodies 22B6D2-hIgG, 47A3E3-hIgG, 100H7D3-hIgG and U1-59 to human and monkey HER3-His proteins was detected by ForteBio (Pall life sciences). The specific method was as follows: The antibody to be tested was diluted to 5µg/ml with PBST (0.02% Tween-20), the human HER3-His protein was serially diluted to 200 nM, 100 nM, 50 nM, 25 nM, 12.50 nM, 6.25 nM, 3.125 nM, 0 nM, and the monkey HER3-His protein was serially diluted to 400 nM, 200 nM, 100 nM, 50 nM, 25 nM, 12.50 nM, 6.25 nM, 0 nM; then the antibody to be tested was captured for 60s with Protein A Sensor (Pall life sciences) in a PBST (0.02% Tween-20) solution, then bound to human or monkey HER3-His protein for 60s, and then dissociated for 180s. The measured results were entered into Data Analysis 11.0 software, by using 1:1 mode, and global fitting; the results were analyzed to obtain the association rate, dissociation rate and affinity constant. The results were shown in Table 5.

**Table 5: Detection of dynamic affinity of anti-human HER3 fully human antibodies to human and monkey HER3-His proteins**

| Detection of dynamic affinity of anti-human HER3 fully human antibodies to human HER3-His | | | |
|---|---|---|---|
| Antibody | KD (M) | kon(1/Ms) | kdis(1/s) |
| 22B6D2-hIgG | 1.11E-08 | 3.57E+05 | 3.97E-03 |
| 47A3E3-hIgG | 2.91E-08 | 3.56E+05 | 1.04E-02 |
| 100H7D3-hIgG | 7.47E-09 | 3.02E+05 | 2.26E-03 |
| U1-59 | 3.64E-09 | 3.24E+05 | 1.18E-03 |

| Detection of dynamic affinity of anti-human HER3 fully human antibodies to monkey HER3-His | | | |
|---|---|---|---|
| Antibody | KD (M) | kon(1/Ms) | kdis(1/s) |
| 22B6D2-hIgG | 4.96E-08 | 1.66E+05 | 8.25E-03 |
| 47A3E3-hIgG | 7.48E-08 | 1.48E+05 | 1.11E-02 |
| 100H7D3-hIgG | 5.44E-09 | 1.70E+05 | 9.26E-04 |
| U1-59 | 1.32E-08 | 1.51E+05 | 2.00E-03 |

### 4.4 Detection of cell affinity of anti-human HER3 fully human antibody

The affinity of the anti-human HER3 fully human antibody to human breast cancer cell T47D (Kangnuotai), human gastric cancer cell NCI-N87 (ATCC), human non-small cell lung cancer cell NCI-H358 (Nanjing Cobioer) was detected by flow cytometer (Beckman, model Cytoflex).

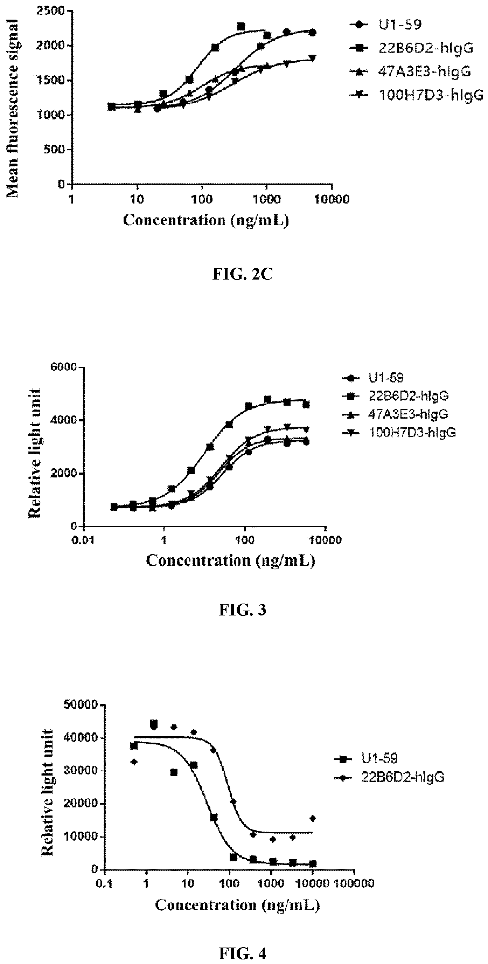

The adherent cells were digested with StemPro^{™} Accutase (Gibco) solution, and counted; an appropriate amount of cells was taken, washed twice with 1xPBS, and resuspended in 1% BSA solution, and then the cells were transferred to a 96-well sharp-bottom plate, 50 µl per well; the candidate antibody was diluted with 1% BSA (starting from 10 µg/ml or 15 µg/ml or 5 µg/ml or 1 µg/ml, with 2-fold or 2.5-fold gradient dilution), and then 50 µl of the diluted antibody was added to the sharp-bottom plate containing the cells, and incubated at 4°C for 40 min; the cells were washed twice with PBS, then 50 µl of the diluted secondary antibody was added to each well, mixed well, and incubated at 4°C for 30 min; the cells were washed twice with PBS, then the cells were resuspended in 400 µl of PBS and detected by flow cytometry. Data processing: the Median PE values were exported, then imported into GraphPad Prism 6 software, and EC50 values were calculated. The results were shown in FIG. 2A, FIG. 2B, FIG. 2C and Table 6. The EC50 values of 22B6D2-hIgG, 47A3E3-hIgG6 and 100H7D3-hIgG were less than that of U1-59, indicating that the affinity on the cells was higher than that of U1-59, in which 22B6D2-hIgG had the highest affinity.

**Table 6: Determination of affinity of anti-human HER3 fully human antibodies to T47D, NCI-N87 and NCI-H358 cells**

| Antibody | T47D (ng/ml) | NCI-N87 (ng/ml) | NCI-H358 (ng/ml) |
|---|---|---|---|
| U1-59 | 590.5 | 536.5 | 328.4 |
| 22B6D2-hIgG | 38.3 | 44.57 | 85.71 |
| 47A3E3-hIgG | 60.1 | 92.34 | 98.94 |
| 100H7D3-hIgG | 202.8 | 217.7 | 290 |

### 4.5 Detection of ADCC activity of anti-human HER3 fully human antibody

Effector cells Jurkat-NFAT/luciferase-CD16a (engineered Jurkat cells stably expressing CD16a and a luciferase reporter gene regulated by NFAT response element, obtained by lentiviral packaging, infection, and stress screening) can activate the expression of luciferase under the coexistence of antibody and target cells, and can emit a fluorescent signal by using ONE-Glo reagent (Promega) as a substrate. The specific method was as follows: Jurkat-NFAT/luciferase-CD16a and CHO-S-hHER3 cells (engineered CHO-S cells stably expressing human HER3, obtained by lentiviral packaging, infection, and stress screening) were collected by centrifugation, resuspended in RPMI 1640+1% FBS medium, adjusted to have a density of 2.0×10⁶/ml and 1.0×10⁶/ml, respectively, added at 50 µL/well to a 96-well plate; the antibody was diluted with RPMI 1640+1% FBS medium (starting from 10 µg/mL, 3-fold dilution, 11 concentration points), added at 50 µL/well to a 96-well plate, and incubated at 37°C for 5 hours; and then 20 µL of One-glo (Promega) detection reagent was added, shaken and mixed; fluorescent signal value was read with a microplate reader, and the results were imported into Graph Prism 6 software to calculate the EC50 values. The results were shown in FIG. 3 and Table 7. The ADCC activity of 22B6D2-hIgG was significantly better than that of U1-59 with 1.86-fold enhancement; while those of 47A3E3-hIgG and 100H7D3-hIgG were comparable to that of U1-59.

**Table 7: Detection of ADCC activity of anti-human HER3 fully human antibody**

| Antibody | EC50 (ng/ml) |
|---|---|
| U1-59 | 27.83 |
| 22B6D2-hIgG | 9.732 |
| 47A3E3-hIgG | 22.32 |
| 100H7D3-hIgG | 25.68 |

### 4.6 Inhibition of ligand-induced AKT phosphorylation with anti-human HER3 fully human antibody

Human breast cancer cells MCF-7 (ATCC) were digested with Tryple and counted, and plated with a growth medium on a 96-well plate at 30000 cells/well/100 µl, incubated overnight at 37°C, 5% CO₂; the growth medium was removed and replaced with serum-free medium with 100 µl/well, for starvation, and the medium was removed after 24 hours. The antibody was serially diluted by serum-free medium (starting from 30 µg/ml, 3-fold gradient, 11 concentration points), and added at 50 µl/well to the plate, incubated in a 37°C incubator for 1 hour. 40 ng/ml NRG1 protein (Sino Biological) was added, 50 µl/well, and returned to the 37°C incubator to stimulate for 20 min. 4% paraformaldehyde was added after removing the liquid, 150 µl/well, and fixed at room temperature for 30 min; anhydrous methanol was added was added after removing the liquid, 150 µl/well, and fixed at room temperature for 20 min; washing was then performed 5 times with PBST (0.2% Tween-20) after removing the liquid, then added with 1% BSA, 200 µl/well, for blocking at room temperature for 2 hours. Akt-P antibody (Cell Signaling Technology) diluted at 1:1000 with 1% BSA was then added, 50 µl/well, placed in a 4°C refrigerator overnight; the primary antibody was removed, washing was performed 3 times with PBST (0.2% Tween-20), and then HRP Goat Anti-rabbit secondary antibody diluted at 1:3000 with 1% BSA was added, 50 µl/well, incubated at room temperature for 1 hour; the secondary antibody was removed, washing was performed 5 times with PBST (0.2% Tween-20); equal volumes of HRP ECL Solution A and Solution B were mixed and balanced, and added at 100 µl/well; the luminescence value was read with a microplate reader. The raw data were imported into Graph Prism 6 to calculate EC50 values. The results were shown in FIG. 4 and Table 8. 22B6D2-hIgG could effectively inhibit ligand-induced AKT phosphorylation.

**Table 8: Determination of inhibition of ligand-induced AKT phosphorylation by anti-human HER3 fully human antibody**

| Antibody | U1-59 | 22B6D2-hIgG |
|---|---|---|
| IC50 (ng/ml) | 28.65 | 91.2 |

### 4.7 Detection of internalization activity of anti-human HER3 fully human antibody

The internalization activity of anti-human HER3 fully human antibody on human breast cancer cell MDA-MB-453 (Kangnuotai), human colorectal cancer cell WIDR (Nanjing Cobioer), human non-small cell lung cancer cell NCI-H358 (Nanjing Cobioer) and human pancreatic cancer cell HPAF-II (Nanjing Cobioer) was detected by Flow cytometry (Beckman, model Cytoflex).

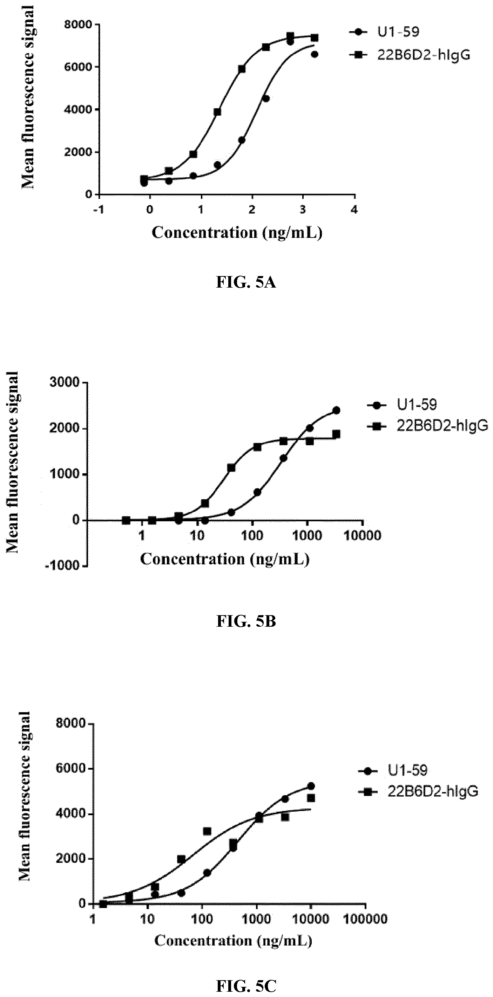

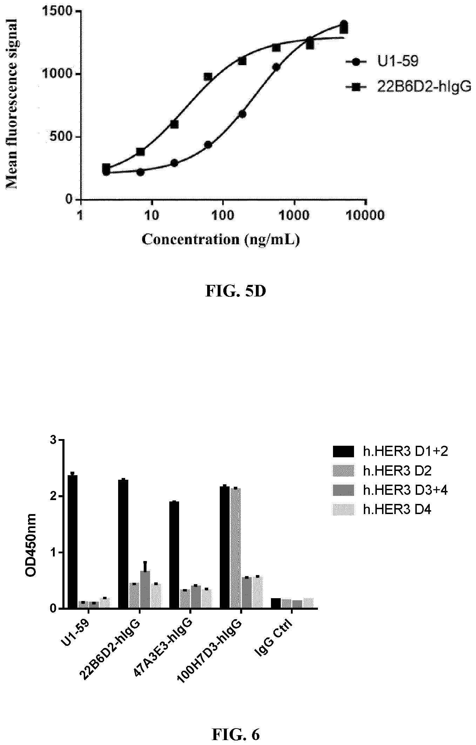

The adherent cells were digested with StemPro^{™} Accutase (Gibco) solution, and counted, and an appropriate amount of cells were taken, washed twice with 1×PBS, resuspended in 1% BSA solution, and then the cells were transferred into a 96-well sharp-bottom plate, 50 µl per well; the candidate antibody was diluted with 1% BSA (starting from 10 µg/ml or 5 µg/ml, with 3-fold or 2.5-fold gradient dilution), then 50 µl of the diluted antibody was added to the sharp-bottom plate containing the cells, incubated at 4°C for 40 minutes; the cells were washed twice with PBS, then added with 50 µl of the diluted secondary antibody Anti-human F(ab')₂ Alexa Fluor 660 to each well, mixed well, and incubated at 4°C for 30 min; the cells were washed twice with PBS, centrifuged at 500g for 3 min, then resuspended with 150 µl of complete medium and allowed to stand at 4°C and 37°C, respectively for incubation for 4 hours. The medium was removed by centrifugation at 500g for 3 minutes, and 100 µl of acid (150 mM NaCl, 0.1 M glycine, adjusted pH to 2.0 with hydrochloric acid) was added to each well to perform resuspending and washing for 2 minutes, then 60 µl of alkali (3 g/L Tris base, 9 g/L NaCl, adjusted pH to 13 with NaOH) was added and mixed for neutralization; the supernatant was removed by centrifugation, and PBS was added for resuspending, and then subjected to detection. The Median APC value was exported, and then imported into GraphPad Prism 6 software to calculate EC50. The results were shown in FIG. 5A, FIG. 5B, FIG. 5C, FIG. 5D and Table 9. The internalization activity of 22B6D2-hIgG was stronger than that of U1-59.

**Table 9: Detection of internalization activity of anti-human HER3 fully human antibody**

| Antibody | MDA-MB-453 (ng/ml) | WIDR (ng/ml) | NCI-358 (ng/ml) | HPAF-II (ng/ml) |
|---|---|---|---|---|
| U1-59 | 121.9 | 333.8 | 449.9 | 282.9 |
| 22B6D2-hIgG | 22.89 | 30.09 | 67.02 | 29.37 |

### 4.8 Detection of binding domain of anti-human HER3 fully human antibody

Human HER3 D1+D2 (aa20~329), human HER3 D2 (aa185~329), human HER3 D3+D4 (aa330~643), human HER3 and D4 (aa496~643) domain proteins were diluted with CBS coating solution to a final concentration of 1 µg/ml, coated at 100 µl/well on a 96-well ELISA plate, and allowed to stand overnight at 4°C; after washing once with PBS, 100 µl of 2% BSA was added to each well, and blocked at 37°C for 2 hours, followed by removing the blocking solution; the biotin-labeled antibody was dilute with 2% BSA to 5 µg/mL, and added at 100 µl/well to the ELISA plate, incubated at 37°C for 2 hours; after washing 3 times with PBST (0.2% Tween-20), 100 µl of the diluted Peroxidase-conjugated Streptavidin (Proteintech) was added to corresponding wells, incubated at 37°C for 1 hour; the plate was then washed 5 times with PBST (0.2% Tween-20), and dried; 100 µl of TMB (InnoReagents) chromogenic substrate was added to each well, reacted at room temperature for 3-5 minutes; 50 µl of 2N H₂SO₄ was added to each well to stop the chromogenic reaction, and OD450 nm absorbance value was read with a microplate reader. The raw data were imported into GraphPad Prism 6 software to make a histogram. The results were shown in FIG. 6. U1-59, 22B6D2-hIgG, and 47A3E3-hIgG mainly bound to the D1 domain of HER3; and 100H7D3-hIgG bound to the D1 and D2 domains of HER3.

### 4.9 Analysis of antigen epitope binding by anti-human HER3 fully human antibody

### 4.9.1 Elisa detection

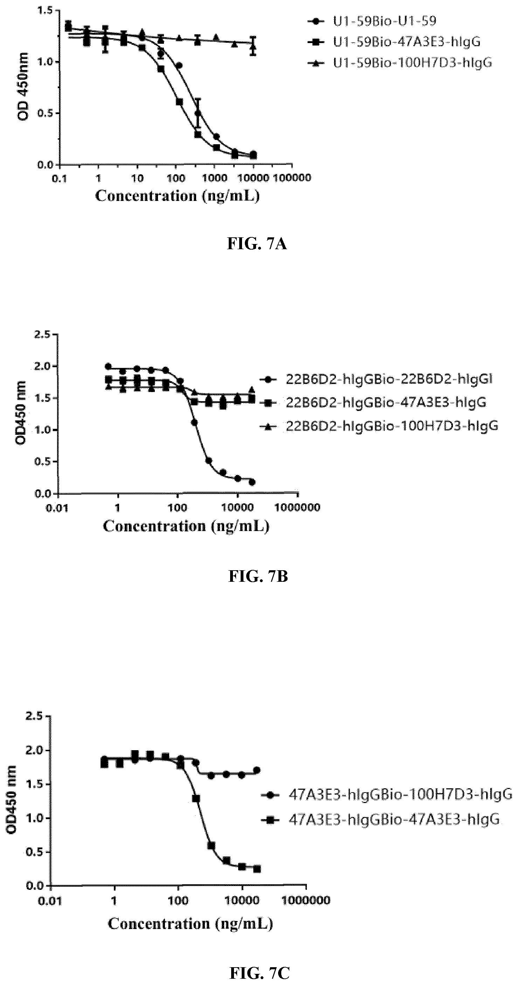

Human HER3 protein was diluted to 1 µg/ml with CBS coating solution, coated at 100 µl/well on a 96-well ELISA plate, and allowed to stand overnight at 4°C; the plate was washed once with PBS, then added with 100 µl of 2% BSA to each well, incubated at 37°C for 2 hours, followed by removing the blocking solution; the human antibody and the control antibody were serially diluted with 2% BSA (starting from 30 µg/ml, 3-fold gradient, 11 concentration points), and added at 50 µl/well to the ELISA plate; then the biotin-labeled U1-59 (U1-59-Bio) was diluted with 2% BSA to 50 ng/ml, the biotin-labeled 22B6D2-hIgG (22B6D2-hIgG-Bio) was diluted with 2% BSA to 3 ng/mL, and the biotin-labeled 47A3E3-hIgG (47A3E3-hIgG-Bio) was diluted with 2% BSA to 15 ng/mL, and added at 50 µl/well to corresponding wells; the serially diluted antibody and biotin-labeled antibodies were mixed well, for incubation at room temperature for 2 hours. The plate was washed with PBST (0.2 % Tween-20) for 3 times, and dried, 100 µl of diluted Peroxidase-conjugated Streptavidin secondary antibody (Proteintech) was added to each well, and incubated at room temperature for 1 hour; the plate was washed 5 times with PBST (0.2% Tween-20), and dried, 100 µl of TMB (InnoReagents) chromogenic substrate was added to each well, reacted at room temperature for 3-5 minutes; 50 µl of 2N H₂SO₄ was added to each well to stop the chromogenic reaction, and OD450 nm absorbance value was read with a microplate reader. The raw data were imported into GraphPad Prism 6 software for nonlinear curve fitting (4-parameter). The results were shown in FIG. 7A, FIG. 7B, and FIG. 7C. The three antibodies 22B6D2-hIgG, 47A3E3-hIgG and 100H7D3-hIgG did not compete with each other; and the epitope of 47A3E3-hIgG was close to that of U1-59.

### 4.9.2 FACS detection

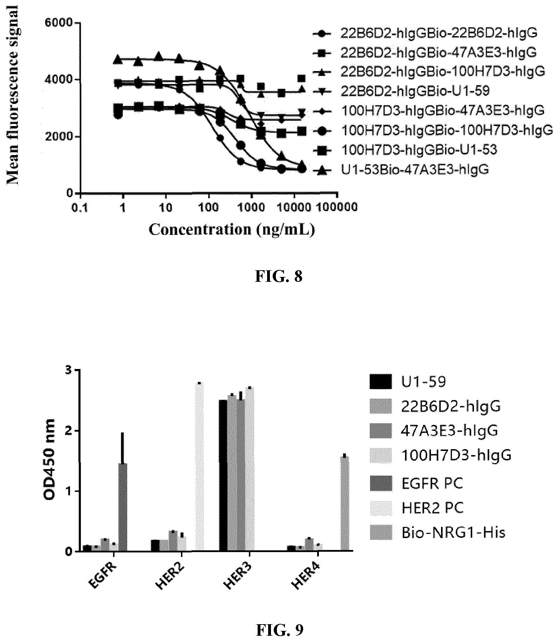

The adherent cells were digested with StemPro^{™} Accutase (Gibco) solution, and counted, and an appropriate amount of the cells were taken and washed once with 1×PBS, resuspended in 1% BSA, and then the cells were transferred to a 96-well sharp-bottom plate, 50 µl per well; the candidate antibody was diluted with 1% BSA (starting from 45 µg/ml, 3-fold gradient, a total of 11 concentration points), and then 50 µl of the diluted antibody was added to the sharp-bottom plate containing the cells; 22B6D2-hIgG-Bio at 75 ng/ml was diluted to final concentration of 25ng/ml, 100H7D3-hIgG-Bio at 600 ng/ml was diluted to final concentration of 200 ng/ml, and U1-59-Bio at 750 ng/ml was diluted to final concentration of 250 ng/ml), and then 50 µl of the diluted antibodies was added to corresponding wells respectively; the cells, the serially diluted antibody and the biotin-labeled antibody with constant concentration as added were mixed, and incubated at 4°C for 40min; the cells were washed twice with PBS, added with 50 µl of the diluted PE Streptavidin secondary antibody (Biolegend) to each well, and incubated at 4°C for 30min; the cells were washed twice with PBS, and finally resuspended in 400 µl of PBS for detection by flow cytometry. Data processing: the Median PE values were exported, and then imported into GraphPad Prism 6 software for nonlinear curve fitting (4-parameter). The results were shown in FIG. 8. The three antibodies 22B6D2-hIgG, 47A3E3-hIgG and 100H7D3-hIgG did not compete with each other; and the epitope of 47A3E3-hIgG was close to that of U1-59.

### 4.10 Detection of specificity of anti-human HER3 fully human antibody

In order to detect the binding of anti-human HER3 fully human antibody to human EGFR (UniProt: P00533), human HER2 (UniProt: P04626), human HER3 (UniProt: P21860), and human HER4 (UniProt: Q15303) of the same family, the human EGFR, human HER2, human HER3 and human HER4 proteins were diluted to 1 µg/ml with CBS coating solution, coated at 100 µl/well on a 96-well ELISA plate, and allowed to stand overnight at 4°C; the ELISA plate was washed once with PBS and dried, then added with 100 µl of 2% BSA to each well, incubated at 37°C for 2 hours, followed by removing the blocking solution; the human antibodies, positive control antibodies against human EGFR, and positive control antibodies against human HER2 were diluted to 10 µg/ml with 2% BSA, respectively, and h.HER4 positive control protein Bio-NRG1-His was diluted to 100 µg/ml, then they were added at 100 µl/well to the ELISA plate, and incubated at 37°C for 2 hours; the plate was washed 3 times with PBST (0.2% Tween-20) and dried, 100 µl of HRP Goat Anti-Human IgG (H+L) (Jackson) was added to each well, and incubated at 37°C for 1 hour. The plate was washed 5 times with PBST (0.2% Tween-20) and dried, 100 µl of TMB (InnoReagents) chromogenic substrate was added to each well, reacted at room temperature for 3-5 minutes, and then 50 µl of 2N H₂SO₄ was added to each well to stop the chromogenic reaction, and OD450 nm absorbance values were read with a microplate reader. The raw data were imported into GraphPad Prism 6 software to make a histogram. The results were shown in FIG. 9. 22B6D2-hIgG, 47A3E3-hIgG and 100H7D3-hIgG all specifically bound to HER3 protein, and basically did not bind to other proteins of the same family.

Although the specific implementation of the present invention has been described in detail, those skilled in the art will understand that: according to all the teachings that have been disclosed, various modifications and changes can be made to the details, and these changes are all within the protection scope of the present invention. The full scope of the present invention is given by the claims appended hereto and any equivalents thereof.

## Claims

1. A antibody or antigen-binding fragment thereof specifically binding to HER3, wherein the antibody or antigen-binding fragment thereof comprises the following complementarity determining regions (CDRs):
(a) CDR-H1, CDR-H2 and CDR-H3 contained in the heavy chain variable region (VH) set forth in SEQ ID NO: 1; and/or, CDR-L1, CDR-L2 and CDR-L3 contained in the light chain variable region (VL) set forth in SEQ ID NO: 2;
(b) CDR-H1, CDR-H2 and CDR-H3 contained in the heavy chain variable region (VH) set forth in SEQ ID NO: 3; and/or, CDR-L1, CDR-L2 and CDR-L3 contained in the light chain variable region (VL) set forth in SEQ ID NO: 4;
(c) CDR-H1, CDR-H2 and CDR-H3 contained in the heavy chain variable region (VH) set forth in SEQ ID NO: 5; and/or, CDR-L1, CDR-L2 and CDR-L3 contained in the light chain variable region (VL) set forth in SEQ ID NO: 6;
(d) CDR-H1, CDR-H2 and CDR-H3 contained in the heavy chain variable region (VH) set forth in SEQ ID NO: 7; and/or, CDR-L1, CDR-L2 and CDR-L3 contained in the light chain variable region (VL) set forth in SEQ ID NO: 8; or
(e) CDR-H1, CDR-H2, and CDR-H3 contained in the following heavy chain variable region (VH), and/or CDR-L1, CDR-L2 and CDR-L3 contained in the following light chain variable region (VL), wherein the heavy chain variable region (VH) and/or the light chain variable region (VL) has at least one CDR containing a mutation compared to the corresponding heavy chain variable region and/or the light chain variable region described in any one of (a) to (d), respectively, and the mutation is a substitution, deletion or addition of one or several amino acids (e.g., a substitution, deletion or addition of 1, 2 or 3 amino acids); preferably, the substitution is a conservative substitution;
preferably, the CDRs are defined according to the IMGT, Kabat or Chothia numbering system.

2. The antibody or antigen-binding fragment thereof according to claim 1, wherein the antibody or antigen-binding fragment thereof comprises:
(1) the following heavy chain variable region (VH) and/or light chain variable region (VL), where the CDRs are defined by the IMGT numbering system:
(1a) a heavy chain variable region (VH) comprising the following 3 CDRs: CDR-H1 having a sequence set forth in SEQ ID NO: 9 or a variant thereof; CDR-H2 having a sequence set forth in SEQ ID NO: 10 or a variant thereof; CDR-H3 having a sequence set forth in SEQ ID NO: 11 or a variant thereof; and/or, a light chain variable region (VL) comprising the following 3 CDRs: CDR-L1 having a sequence set forth in SEQ ID NO: 18 or a variant thereof; CDR-L2 having a sequence set forth in SEQ ID NO: 19 or a variant thereof; CDR-L3 having a sequence set forth in SEQ ID NO: 20 or a variant thereof;
(1b) a heavy chain variable region (VH) comprising the following 3 CDRs: CDR-H1 having a sequence set forth in SEQ ID NO: 24 or a variant thereof; CDR-H2 having a sequence set forth in SEQ ID NO: 25 or a variant thereof; CDR-H3 having a sequence set forth in SEQ ID NO: 26 or a variant thereof; and/or, a light chain variable region (VL) comprising the following 3 CDRs: CDR-L1 having a sequence set forth in SEQ ID NO: 67 or a variant thereof; CDR-L2 having a sequence set forth in SEQ ID NO: 68 or a variant thereof; CDR-L3 having a sequence set forth in SEQ ID NO: 69 or a variant thereof;
(1c) a heavy chain variable region (VH) comprising the following 3 CDRs: CDR-H1 having a sequence set forth in SEQ ID NO: 37 or a variant thereof; CDR-H2 having a sequence set forth in SEQ ID NO: 38 or a variant thereof; CDR-H3 having a sequence set forth in SEQ ID NO: 39 or a variant thereof; and/or, a light chain variable region (VL) comprising the following 3 CDRs: CDR-L1 having a sequence set forth in SEQ ID NO: 32 or a variant thereof; CDR-L2 having a sequence set forth in SEQ ID NO: 33 or a variant thereof; CDR-L3 having a sequence set forth in SEQ ID NO: 34 or a variant thereof; or,
(1d) a heavy chain variable region (VH) comprising the following 3 CDRs: CDR-H1 having a sequence set forth in SEQ ID NO: 45 or a variant thereof; CDR-H2 having a sequence set forth in SEQ ID NO: 46 or a variant thereof; CDR-H3 having a sequence set forth in SEQ ID NO: 47 or a variant thereof; and/or, a light chain variable region (VL) comprising the following 3 CDRs: CDR-L1 having a sequence set forth in SEQ ID NO: 53 or a variant thereof; CDR-L2 having a sequence set forth in SEQ ID NO: 33 or a variant thereof; CDR-L3 having a sequence set forth in SEQ ID NO: 54 or a variant thereof;
or,
(2) the following heavy chain variable region (VH) and/or light chain variable region (VL), where the CDRs are defined according to the Kabat numbering system:
(2a) a heavy chain variable region (VH) comprising the following 3 CDRs: CDR-H1 having a sequence set forth in SEQ ID NO: 12 or a variant thereof; CDR-H2 having a sequence set forth in SEQ ID NO: 13 or a variant thereof; CDR-H3 having a sequence set forth in SEQ ID NO: 14 or a variant thereof; and/or, a light chain variable region (VL) comprising the following 3 CDRs: CDR-L1 having a sequence set forth in SEQ ID NO: 21 or a variant thereof; CDR-L2 having a sequence set forth in SEQ ID NO: 22 or a variant thereof; CDR-L3 having a sequence set forth in SEQ ID NO: 23 or a variant thereof;
(2b) a heavy chain variable region (VH) comprising the following 3 CDRs: CDR-H1 having a sequence set forth in SEQ ID NO: 27 or a variant thereof; CDR-H2 having a sequence set forth in SEQ ID NO: 28 or a variant thereof; CDR-H3 having a sequence set forth in SEQ ID NO: 29 or a variant thereof; and/or, a light chain variable region (VL) comprising the following 3 CDRs: CDR-L1 having a sequence set forth in SEQ ID NO: 70 or a variant thereof; CDR-L2 having a sequence set forth in SEQ ID NO: 71 or a variant thereof; CDR-L3 having a sequence set forth in SEQ ID NO: 69 or a variant thereof;
(2c) a heavy chain variable region (VH) comprising the following 3 CDRs: CDR-H1 having a sequence set forth in SEQ ID NO: 40 or a variant thereof; CDR-H2 having a sequence set forth in SEQ ID NO: 41 or a variant thereof; CDR-H3 having a sequence set forth in SEQ ID NO: 42 or a variant thereof; and/or, a light chain variable region (VL) comprising the following 3 CDRs: CDR-L1 having a sequence set forth in SEQ ID NO: 35 or a variant thereof; CDR-L2 having a sequence set forth in SEQ ID NO: 36 or a variant thereof; CDR-L3 having a sequence set forth in SEQ ID NO: 34 or a variant thereof; or
(2d) a heavy chain variable region (VH) comprising the following 3 CDRs: CDR-H1 having a sequence set forth in SEQ ID NO: 48 or a variant thereof; CDR-H2 having a sequence set forth in SEQ ID NO: 49 or a variant thereof; CDR-H3 having a sequence set forth in SEQ ID NO: 50 or a variant thereof; and/or, a light chain variable region (VL) comprising the following 3 CDRs: CDR-L1 having a sequence set forth in SEQ ID NO: 72 or a variant thereof; CDR-L2 having a sequence set forth in SEQ ID NO: 36 or a variant thereof; CDR-L3 having a sequence set forth in SEQ ID NO: 54 or a variant thereof;
or,
(3) the following heavy chain variable region (VH) and/or light chain variable region (VL), where the CDRs are defined by the Chothia numbering system:
(3a) a heavy chain variable region (VH) comprising the following 3 CDRs: CDR-H1 having a sequence set forth in SEQ ID NO: 15 or a variant thereof; CDR-H2 having a sequence set forth in SEQ ID NO: 16 or a variant thereof; CDR-H3 having a sequence set forth in SEQ ID NO: 17 or a variant thereof; and/or, a light chain variable region (VL) comprising the following 3 CDRs: CDR-L1 having a sequence set forth in SEQ ID NO: 21 or a variant thereof; CDR-L2 having a sequence set forth in SEQ ID NO: 22 or a variant thereof; CDR-L3 having a sequence set forth in SEQ ID NO: 23 or a variant thereof;
(3b) a heavy chain variable region (VH) comprising the following 3 CDRs: CDR-H1 having a sequence set forth in SEQ ID NO: 30 or a variant thereof; CDR-H2 having a sequence set forth in SEQ ID NO: 31 or a variant thereof; CDR-H3 having a sequence set forth in SEQ ID NO: 29 or a variant thereof; and/or, a light chain variable region (VL) comprising the following 3 CDRs: CDR-L1 having a sequence set forth in SEQ ID NO: 70 or a variant thereof; CDR-L2 having a sequence set forth in SEQ ID NO: 71 or a variant thereof; CDR-L3 having a sequence set forth in SEQ ID NO: 69 or a variant thereof;
(3c) a heavy chain variable region (VH) comprising the following 3 CDRs: CDR-H1 having a sequence set forth in SEQ ID NO: 43 or a variant thereof; CDR-H2 having a sequence set forth in SEQ ID NO: 44 or a variant thereof; CDR-H3 having a sequence set forth in SEQ ID NO: 42 or a variant thereof; and/or, a light chain variable region (VL) comprising the following 3 CDRs: CDR-L1 having a sequence set forth in SEQ ID NO: 35 or a variant thereof; CDR-L2 having a sequence set forth in SEQ ID NO: 36 or a variant thereof; CDR-L3 having a sequence set forth in SEQ ID NO: 34 or a variant thereof; or
(3d) a heavy chain variable region (VH) comprising the following 3 CDRs: CDR-H1 having a sequence set forth in SEQ ID NO: 51 or a variant thereof; CDR-H2 having a sequence set forth in SEQ ID NO: 52 or a variant thereof; CDR-H3 having a sequence set forth in SEQ ID NO: 50 or a variant thereof; and/or, a light chain variable region (VL) comprising the following 3 CDRs: CDR-L1 having a sequence set forth in SEQ ID NO: 72 or a variant thereof; CDR-L2 having a sequence set forth in SEQ ID NO: 36 or a variant thereof; CDR-L3 having a sequence set forth in SEQ ID NO: 54 or a variant thereof;
wherein, the variant described in any one of (1a) to (1d), (2a) to (2d), (3a) to (3d) has a substitution, deletion or addition of one or several amino acids (e.g., a substitution, deletion or addition of 1, 2 or 3 amino acids) compared to the sequence from which it is derived; preferably, the substitution is a conservative substitution.

3. The antibody or antigen-binding fragment thereof according to claim 1 or 2, wherein the antibody or antigen-binding fragment thereof comprises:
(4a) a VH comprising the sequence set forth in SEQ ID NO: 1 or a variant thereof and/or a VL comprising the sequence set forth in SEQ ID NO: 2 or a variant thereof;
(4b) a VH comprising the sequence set forth in SEQ ID NO: 3 or a variant thereof and/or a VL comprising the sequence set forth in SEQ ID NO: 4 or a variant thereof;
(4c) a VH comprising a sequence as set forth in SEQ ID NO: 5 or a variant thereof and/or a VL comprising the sequence set forth in SEQ ID NO: 6 or a variant thereof; or
(4d) a VH comprising the sequence set forth in SEQ ID NO: 7 or a variant thereof and/or a VL comprising the sequence set forth in SEQ ID NO: 8 or a variant thereof;
wherein the variant has a sequence identity of at least 80%, at least 85%, at least 90%, at least 91%, at least 92%, at least 93%, at least 94%, at least 95%, at least 96%, at least 97%, at least 98%, at least 99%, or 100% compared to the sequence from which it is derived, or has a substitution, deletion, or addition of one or several amino acids (e.g., a substitution, deletion, or addition of 1, 2, 3, 4, or 5 amino acids) compared to the sequence from which it is derived; preferably, the substitution is a conservative substitution.

4. The antibody or antigen-binding fragment thereof according to any one of claims 1-3, wherein the antibody or antigen-binding fragment thereof is a murine antibody, a chimeric antibody, a humanized antibody or a fully human antibody.

5. The antibody or antigen-binding fragment thereof according to any one of claims 1-4, wherein the antibody or antigen-binding fragment thereof further comprises a constant region from or derived from a human immunoglobulin;
preferably, the heavy chain of the antibody or antigen-binding fragment thereof comprises a heavy chain constant region from or derived from a human immunoglobulin (e.g., IgG1, IgG2, IgG3 or IgG4); preferably, the antibody or antigen-binding fragment thereof comprises a wild-type Fc region, or comprises a mutated or chemically modified Fc region, which has an altered effector function compared to a wild-type Fc region;
preferably, the antibody or antigen-binding fragment thereof has a light chain comprising a light chain constant region from or derived from a human immunoglobulin (e.g., κ or λ);
preferably, the antibody or antigen-binding fragment thereof comprises a heavy chain constant region (CH) as set forth in SEQ ID NO: 63 or a variant thereof, wherein the variant has a conservative substitution of up to 20 amino acids (e.g., a conservative substitution of up to 15, up to 10, or up to 5 amino acids; for example a conservative substitution of 1, 2, 3, 4 or 5 amino acids) compared to SEQ ID NO: 63;
preferably, the antibody or antigen-binding fragment thereof comprises a light chain constant region (CL) as set forth in SEQ ID NO: 65 or a variant thereof, wherein the variant has a conservative substitution of up to 20 amino acids (e.g., a conservative substitution of up to 15, up to 10, or up to 5 amino acids; for example, a conservative substitution of 1, 2, 3, 4 or 5 amino acids) compared to SEQ ID NO: 65;
more preferably, the antibody or antigen-binding fragment thereof comprises a heavy chain constant region (CH) as set forth in SEQ ID NO:63 and a light chain constant region (CL) as set forth in SEQ ID NO:65.

6. The antibody or antigen-binding fragment thereof according to any one of claims 1-5, wherein the antibody or antigen-binding fragment thereof comprises:
(1) a heavy chain comprising a VH set forth in SEQ ID NO: 1 and a heavy chain constant region (CH) set forth in SEQ ID NO: 63, and/or, a light chain comprising a VL set forth in SEQ ID NO: 2 and a light chain constant region (CL) set forth in SEQ ID NO: 65;
(2) a heavy chain comprising a VH set forth in SEQ ID NO: 3 and a heavy chain constant region (CH) set forth in SEQ ID NO: 63, and/or, a light chain comprising a VL set forth in SEQ ID NO: 4 and a light chain constant region (CL) set forth in SEQ ID NO: 65;
(3) a heavy chain comprising a VH set forth in SEQ ID NO: 5 and a heavy chain constant region (CH) set forth in SEQ ID NO: 63, and/or, a light chain comprising a VL set forth in SEQ ID NO: 6 and a light chain constant region (CL) set forth in SEQ ID NO: 65;
or,
(4) a heavy chain comprising a VH set forth in SEQ ID NO: 7 and a heavy chain constant region (CH) set forth in SEQ ID NO: 63, and/or, a light chain comprising a VL set forth in SEQ ID NO: 8 and a light chain constant region (CL) set forth in SEQ ID NO: 65.

7. The antibody or antigen-binding fragment thereof according to any one of claims 1-6, wherein the antibody or antigen-binding fragment thereof is selected from the group consisting of ScFv, Fab, Fab', Fab'-SH, (Fab')₂, Fv fragment, disulfide bond-linked Fv(dsFv), diabody, bispecific antibody and multispecific antibody.

8. The antibody or antigen-binding fragment thereof according to any one of claims 1-7, wherein the antibody or antigen-binding fragment thereof bears a label; preferably, the antibody or antigen-binding fragment thereof bears a detectable label, such as enzyme (e.g., horseradish peroxidase), radionuclide, fluorescent dye, luminescent substance (e.g., chemiluminescent substance), or biotin.

9. The antibody or antigen-binding fragment thereof according to any one of claims 1-8, wherein the antibody or antigen-binding fragment thereof has a feature selected from the group consisting of:
(1) binding to HER3 (e.g., human or monkey HER3) with an EC50 of less than about 100 ng/mL, for example less than about 80 ng/mL, 50 ng/mL, 20 ng/mL, 15 ng/mL, 14 ng/mL, 13 ng/mL, 12 ng/mL, 11 ng/mL, 10 ng/mL, 9 ng/mL, 8 ng/mL, 7 ng/mL, 6 ng/mL, 5 ng/mL, 4 ng/mL or less; preferably, the EC50 is determined by ELISA;
(2) binding to HER3 (e.g., human or monkey HER3) with a KD of less than about 100 nM, for example less than about 90 nM, 80 nM, 70 nM, 60 nM, 50 nM, 40 nM, 30 nM, 20 nM, 15 nM, 10 nM, 5 nM or lower; preferably, the KD is determined by biolayer interferometry (BLI) (e.g., ForteBio Octet^{®});
(3) not binding or substantially not binding to EGFR, HER2 and/or HER4; for example, determined by ELISA;
(4) having ADCC activity, such as inducing and killing HER3-expressing cells (e.g., tumor cells) through ADCC;
(5) inhibiting HER3-mediated signaling, such as inhibiting ligand-induced AKT phosphorylation, and/or inhibiting PI3K/AKT signaling;
(6) inducing HER3 internalization, for example, determined by flow cytometry;
(7) inhibiting cell (e.g., tumor cell) proliferation; and/or
(8) inhibiting tumor growth.

10. An isolated nucleic acid molecule, which comprises a nucleotide sequence encoding the antibody or antigen-binding fragment thereof according to any one of claims 1-9, its heavy chain and/or light chain, or its heavy chain variable region and/or light chain variable region.

11. The isolated nucleic acid molecule according to claim 10, which comprises a nucleic acid molecule encoding an antibody heavy chain variable region, and/or a nucleic acid molecule encoding an antibody light chain variable region, wherein:
(5a) the nucleic acid molecule encoding the antibody heavy chain variable region comprises: (i) a nucleotide sequence set forth in SEQ ID NO: 55, (ii) a sequence substantially identical to SEQ ID NO: 55 (e.g., a sequence having a sequence identity of at least about 85%, 90%, 95%, 99% or more, or a sequence having a substitution of one or more nucleotides, compared to SEQ ID NO: 55), or (iii) a degenerate sequence of the above (i) or (ii); and/or, the nucleic acid molecule encoding the antibody light chain variable region comprises: (iv) a nucleotide sequence set forth in SEQ ID NO: 56, (v) a sequence substantially identical to SEQ ID NO: 56 (e.g., a sequence having a sequence identity of at least about 85%, 90%, 95%, 99% or more, or a sequence having a substitution of one or more nucleotides, compared to SEQ ID NO: 56), or (vi) a degenerate sequence of the above (iv) or (v);
or
(5b) the nucleic acid molecule encoding the antibody heavy chain variable region comprises: (i) a nucleotide sequence set forth in SEQ ID NO: 57, (ii) a sequence substantially identical to SEQ ID NO: 57 (e.g., a sequence having a sequence identity of at least about 85%, 90%, 95%, 99% or more, or a sequence having a substitution of one or more nucleotides, compared to SEQ ID NO: 57), or (iii) a degenerate sequence of the above (i) or (ii); and/or, the nucleic acid molecule encoding the antibody light chain variable region comprises: (iv) a nucleotide sequence set forth in SEQ ID NO: 58, (v) a sequence substantially identical to SEQ ID NO: 58 (e.g., a sequence having a sequence identity of at least about 85%, 90%, 95%, 99% or more, or a sequence having a substitution of one or more nucleotides, compared to SEQ ID NO: 58), or (vi) a degenerate sequence of the above (iv) or (v);
or
(5c) the nucleic acid molecule encoding the antibody heavy chain variable region comprises: (i) a nucleotide sequence set forth in SEQ ID NO: 59, (ii) a sequence substantially identical to SEQ ID NO: 59 (e.g., a sequence having a sequence identity of at least about 85%, 90%, 95%, 99% or more, or a sequence having a substitution of one or more nucleotides, compared to SEQ ID NO: 59), or (iii) a degenerate sequence of the above (i) or (ii); and/or, the nucleic acid molecule encoding the antibody light chain variable region comprises: (iv) a nucleotide sequence set forth in SEQ ID NO: 60, (v) a sequence substantially identical to SEQ ID NO: 60 (e.g., a sequence having a sequence identity of at least about 85%, 90%, 95%, 99% or more, or a sequence having a substitution of one or more nucleotides, compared to SEQ ID NO: 60), or (vi) a degenerate sequence of the above (iv) or (v);
or
(5d) the nucleic acid molecule encoding the antibody heavy chain variable region comprises: (i) a nucleotide sequence set forth in SEQ ID NO: 61, (ii) a sequence substantially identical to SEQ ID NO: 61 (e.g., a sequence having a sequence identity of at least about 85%, 90%, 95%, 99% or more, or a sequence having a substitution of one or more nucleotides, compared to SEQ ID NO: 61), or (iii) a degenerate sequence of the above (i) or (ii); and/or, the nucleic acid molecule encoding the antibody light chain variable region comprises: (iv) a nucleotide sequence set forth in SEQ ID NO: 62, (v) a sequence substantially identical to SEQ ID NO: 62 (e.g., a sequence having a sequence identity of at least about 85%, 90%, 95%, 99% or more, or a sequence having a substitution of one or more nucleotides, compared to SEQ ID NO: 62), or (vi) a degenerate sequence of the above (iv) or (v).

12. A vector, which comprises the nucleic acid molecule according to any one of claims 10-11; preferably, the vector is a cloning vector or an expression vector.

13. A host cell, which comprises the nucleic acid molecule according to any one of claims 10-11 or the vector according to claim 12.

14. A method for preparing the antibody or antigen-binding fragment thereof according to any one of claims 1-9, comprising culturing the host cell according to claim 13 under conditions that allow expression of the antibody or antigen-binding fragment thereof, and recovering the antibody or antigen-binding fragment thereof from a culture of the cultured host cell.

15. A conjugate, which comprises the antibody or antigen-binding fragment thereof according to any one of claims 1-9 and a conjugating moiety linked thereto;
preferably, the conjugating moiety is selected from the group consisting of detectable label (e.g., radioisotope, fluorescent substance, luminescent substance, colored substance or enzyme) or therapeutic agent (e.g., cytotoxic agent, cytokine, toxin or radionuclide).

16. A multispecific antibody, which comprises the antibody or antigen-binding fragment thereof according to any one of claims 1-9;
preferably, the multispecific antibody comprises the antibody or antigen-binding fragment thereof according to any one of claims 1-9 as a first antigen-binding domain, and further comprises at least one second antigen-binding domain directed against other target;
preferably, the multispecific antibody is a bispecific antibody, a trispecific antibody or a tetraspecific antibody.

17. A chimeric antigen receptor, which comprises the antibody or antigen-binding fragment thereof (e.g., ScFv) according to any one of claims 1-9, a transmembrane domain, and one or more intracellular T cell signaling domains.

18. A pharmaceutical composition, which comprises the antibody or antigen-binding fragment thereof according to any one of claims 1-9, or the isolated nucleic acid molecule according to claim 10 or 11, or the vector according to claim 12, or the host cell according to claim 13, or the conjugate according to claim 15, or the multispecific antibody according to claim 16, or the chimeric antigen receptor according to claim 17 or a host cell expressing the chimeric antigen receptor, and a pharmaceutically acceptable carrier and/or excipient;
preferably, the pharmaceutical composition further comprises an additional pharmaceutically active agent;
preferably, the additional pharmaceutically active agent is a drug with antitumor activity;
preferably, the additional pharmaceutically active agent is selected from the group consisting of: EGFR inhibitor, HER2 inhibitor, HER3 inhibitor, HER4 inhibitor, IGFR-1 inhibitor, mTOR inhibitor, PI3 kinase inhibitor, c-met or VEGF inhibitor, chemotherapeutic agent, or any combination thereof;
preferably, the antibody or antigen-binding fragment thereof and the additional pharmaceutically active agent are provided as separate components or as mixed components.

19. A diagnostic or therapeutic kit, which comprises the antibody or antigen-binding fragment thereof according to any one of claims 1-9, or the isolated nucleic acid molecule according to claim 10 or 11, or the vector according to claim 12, or the host cell according to claim 13, or the conjugate according to claim 15, or the multispecific antibody according to claim 16, or the chimeric antigen receptor according to claim 17 or a host cell expressing the chimeric antigen receptor, or the pharmaceutical composition according to claim 18, and optionally an instruction for use and/or an administration device.

20. Use of the antibody or antigen-binding fragment thereof according to any one of claims 1-9, or the isolated nucleic acid molecule according to claim 10 or 11, or the vector according to claim 12, or the host cell according to claim 13, or the conjugate according to claim 15, or the multispecific antibody according to claim 16, or the chimeric antigen receptor according to claim 17 or a host cell expressing the chimeric antigen receptor, or the pharmaceutical composition according to claim 18 in the manufacture of a medicament for inhibiting the proliferation of a cell (e.g., a cell expressing HER3, such as a tumor cell), or prevention and/or treatment and/or adjuvant treatment of a tumor;
preferably, the antibody or antigen-binding fragment thereof, the isolated nucleic acid molecule, the vector, the host cell, the conjugate, the multispecific antibody, or the pharmaceutical composition is administered, for example simultaneously, separately or sequentially, in combination with an additional pharmaceutically active agent, the IMGT, Kabat or Chothia numbering system;
preferably, the additional pharmaceutically active agent is a drug with antitumor activity;
preferably, the additional pharmaceutically active agent is selected from the group consisting of: EGFR inhibitor, HER2 inhibitor, HER3 inhibitor, HER4 inhibitor, IGFR-1 inhibitor, mTOR inhibitor, PI3 kinase inhibitor, c-met or VEGF inhibitor, chemotherapeutic agent, or any combination thereof.

21. The use according to claim 20, wherein the tumor is a HER3-positive tumor;
preferably, the tumor is selected from the group consisting of breast cancer, gastric cancer, lung cancer (e.g., non-small cell lung cancer), colorectal cancer, pancreatic cancer, head and neck squamous cell carcinoma, melanoma, ovarian cancer, prostate cancer, liver cancer, kidney cancer, bladder cancer, or any combination thereof.

22. A method for inhibiting cell proliferation, comprising contacting the cell with the antibody or antigen-binding fragment thereof according to any one of claims 1-9, or the isolated nucleic acid molecule according to claim 10 or 11, or the vector according to claim 12, or the host cell according to claim 13, or the conjugate according to claim 15, or the multispecific antibody according to claim 16, or the chimeric antigen receptor according to claim 17 or a host cell expressing the chimeric antigen receptor, or the pharmaceutical composition according to claim 18;
preferably, the cell is a HER3-expressing cell, such as a tumor cell.

23. A method for prevention and/or treatment and/or adjuvant treatment of a tumor in a subject, the method comprising administering to the subject in need thereof an effective amount of the antibody or antigen-binding fragment thereof according to any one of claims 1-9, or the isolated nucleic acid molecule according to claim 10 or 11, or the vector according to claim 12, or the host cell according to claim 13, or the conjugate according to claim 15, or the multispecific antibody according to claim 16, or the chimeric antigen receptor according to claim 17 or a host cell expressing the chimeric antigen receptor, or the pharmaceutical composition according to claim 18.

24. The method according to claim 23, which further comprises administering to the subject a second therapy, wherein the second therapy is selected from the group consisting of surgery, chemotherapy, radiotherapy, immunotherapy, gene therapy, DNA therapy, RNA therapy, nano-therapy, virotherapy, adjuvant therapy, and any combination thereof;
optionally, the second therapy may be applied simultaneously, separately or sequentially with the method according to claim 23.

25. The method according to claim 23 or 24, wherein the tumor is a HER3-positive tumor;
preferably, the tumor is selected from the group consisting of breast cancer, gastric cancer, lung cancer (e.g., non-small cell lung cancer), colorectal cancer, pancreatic cancer, head and neck squamous cell carcinoma, melanoma, ovarian cancer, prostate cancer, liver cancer, kidney cancer, bladder cancer, or any combination thereof.

26. A method for detecting the presence or level of HER3 in a sample, comprising contacting the sample with the antibody or antigen-binding fragment thereof according to any one of claims 1-9 under conditions that allow the formation of a complex between the antibody or antigen-binding fragment thereof and HER3, and detecting the formation of the complex;
preferably, the method is used for the diagnosis of a tumor, for example a HER3-positive tumor, such as breast cancer, gastric cancer, lung cancer (e.g., non-small cell lung cancer), colorectal cancer, pancreatic cancer, head and neck squamous cell carcinoma, melanoma, ovarian cancer, prostate cancer, liver cancer, kidney cancer, bladder cancer, or any combination thereof;
preferably, the method comprises detecting an expression level of HER3 in a test sample from the subject, and comparing the expression level with a reference value, wherein an increase in the expression level compared to the reference value is indicative of a tumor.

27. Use of the antibody or antigen-binding fragment thereof according to any one of claims 1-9, or the isolated nucleic acid molecule according to claim 10 or 11, or the vector according to claim 12, or the host cell according to claim 13, or the conjugate according to claim 15, or the multispecific antibody according to claim 16 in the manufacture of a kit, wherein the kit is used for detecting the presence or level of HER3 in a sample and/or for diagnosing a tumor;
preferably, the tumor is a HER3-positive tumor;
preferably, the tumor is selected from the group consisting of breast cancer, gastric cancer, lung cancer (e.g., non-small cell lung cancer), colorectal cancer, pancreatic cancer, head and neck squamous cell carcinoma, melanoma, ovarian cancer, prostate cancer, liver cancer, kidney cancer, bladder cancer, or any combination thereof.
